# EUROPEAN PATENT APPLICATION

(11) **EP 4 620 383 A1**
(43) Date of publication of application: **24.09.2025**
(21) Application number: 25165427.3
(22) Date of filing: 21.03.2025
(51) Int. Cl.: A61B 5/00, A61B 5/30

(54) **ECG MONITORING SYSTEM HAVING WIRELESS CHARGING AND COMMUNICATION**

(30) Priority: 22.03.2024 US 202463568658 P; 16.05.2024 US 202463648247 P
(71) Applicant: Welch Allyn, Inc., Skaneateles Falls, NY 13153-0220 (US)
(72) Inventor: DO, Tiffany, Skaneateles Falls, 13153-0220 (US); VILLARREAL, David, Skaneateles Falls, 13153-0220 (US); REVINSKI, Alexey, Skaneateles Falls, 13153-0220 (US); MARTIN, Scott Andrew, Skaneateles Falls, 13153-0220 (US); ROBERTS, Chris R., Skaneateles Falls, 13153-0220 (US)
(74) Representative: Reddie & Grose LLP

(57) **Abstract**

A biosignal monitoring system comprises a wireless signal acquisition device, a dock, and a positioning assembly. The wireless signal acquisition device is configured to acquire biosignals of a patient. The wireless signal acquisition device includes a housing and a device charging unit positioned inside of the housing and including a rechargeable battery and a power receiver. The dock is formed to include a first acquisition-device receiver to receive the wireless signal acquisition device therein and includes a dock charging unit. The positioning assembly is configured to mechanically and magnetically align the wireless signal acquisition device within the first acquisition-device receiver of the dock so that the power receiver of the wireless signal acquisition device is aligned with the dock charging unit of the dock and the rechargeable battery is wirelessly recharged.

## Description

The present disclosure relates to monitoring systems, and particularly, to biosignal monitoring systems. More particularly, the present disclosure relates to biosignal monitoring systems including a wireless signal acquisition device and a dock.

Generally, biosignal monitoring systems include wired signal acquisition devices requiring a cable connection to a power source. Cable connections may limit a patient's range of motion and/or obstruct a technician's range of motion. Wired signal acquisition devices may require electrical isolation of the patient from the system via an isolation barrier, which may add cost, may increase the size of the device, and may require additional safety testing for the device.

Some biosignal monitoring systems include wireless signal acquisition devices; however, these devices typically include primary batteries or swappable secondary batteries. The technician may be required to continuously purchase and replace primary batteries. Additionally, in order to replace primary batteries, the device may include a battery door, which may be an additional access point for liquid ingress and may hinder cleaning of the device. Thus, wireless signal acquisition devices including a captive secondary battery would be appreciated by patients and technicians. However, a captive secondary battery may require the wireless signal acquisition device to connect to a charging power source via a wired cable, which may result in similar problems to the wired signal acquisition device. The secondary battery may be wirelessly recharged using a short-range inductive wireless charging protocol, such as Qi, AirFuel Alliance, or similar technology. However, using Qi in a low-power, small form-factor system may be costly, may generate too much heat within the device, and may be inefficient at transferring low amounts of power to the device. Further, utilizing Qi protocol may require cross-platform certification testing, and the system may be forced to accommodate and work with external medical or consumer devices not included in the system. Therefore, wireless signal acquisition devices with a custom wireless recharging technology implementation may be beneficial for lowering total solution cost, power transfer efficiency, improved heat dissipation, and incompatibility with consumer-grade charging technologies.

An apparatus, system, or method may comprise one or more of the following features, alone or in any combination:

According to the present disclosure, a biosignal monitoring system may be provided. The biosignal monitoring system may comprise a wireless signal acquisition device, a dock, and a positioning assembly. The wireless signal acquisition device may be configured to acquire biosignals of a patient. The wireless signal acquisition device may include a housing and a device charging unit positioned inside of the housing. The device charging unit may include a rechargeable battery configured to provide power to the wireless signal acquisition device and a power receiver configured to recharge the rechargeable battery. The dock may be configured to receive a power cable therein. The dock may be formed to include a first acquisition-device receiver to receive the wireless signal acquisition device therein. The dock may include a dock charging unit positioned inside of the dock. The positioning assembly may be configured to mechanically and magnetically align the wireless signal acquisition device within the first acquisition-device receiver of the dock to removably position the wireless signal acquisition device in the first acquisition-device receiver so that the power receiver of the wireless signal acquisition device and the dock charging unit of the dock are aligned for wireless recharging of the rechargeable battery.

Optionally, the wireless signal acquisition device may be configured to engage wirelessly with the dock and the dock may be configured to engage wirelessly with the wireless signal acquisition device. The wireless signal acquisition device may include a wireless data transmitter. The wireless data transmitter may be configured to transmit biosignal data and power feedback data to the dock. The dock may include a wireless data receiver. The wireless data receiver may be configured to receive the biosignal data and the power feedback data from the wireless data transmitter of the wireless signal acquisition device.

Further optionally, the positioning assembly may comprise a magnet coupled to the dock and a ferromagnetic metal component coupled to the housing of the wireless signal acquisition device. The magnet and the ferromagnetic metal component may be positioned such that the magnet and the ferromagnetic metal component are aligned and attracted to one another while the wireless signal acquisition device is positioned in the dock. The magnet may be arranged inside of the dock and the ferromagnetic metal component may be arranged inside of the housing of the wireless signal acquisition device. The detected biosignals may be electrocardiogram signals.

If desired, the biosignal monitoring system may further comprise a lead set including a first end removably coupled to the housing of the wireless signal acquisition device and a second end opposite the first end and including a plurality of wire leads configured to be coupled to electrodes positioned on the patient. The dock may be formed to include a lead set holder configured to extend around at least a portion of the lead set while the wireless signal acquisition device is received in the dock to maintain a position of the lead set relative to the dock. The second end may provide a 12 lead diagnostic signal. The second end may provide a 15 lead diagnostic signal. The second end may provide a 5 lead diagnostic signal. The second end may provide a 3 lead diagnostic signal.

Alternatively, the wireless signal acquisition device may include a first button positioned on the housing and configured to be actuated to start an electrocardiogram exam. The wireless signal acquisition device may include a biosignal status indicator extending around a perimeter of the first button and configured to illuminate to indicate acquisition of the biosignals during the electrocardiogram exam. The wireless signal acquisition device may include a second button positioned on the housing and configured to be actuated to start a rhythm exam. The wireless signal acquisition device may include a biosignal status indicator extending around a perimeter of the second button and configured to illuminate to indicate acquisition of the biosignals during the rhythm exam. The wireless signal acquisition device may include a third button positioned on the housing and configured to be actuated to power on, to power off, and to wake up the wireless signal acquisition device.

Additionally, the wireless signal acquisition device may include a charge status indicator positioned on the housing. The charge status indicator may be configured to illuminate to indicate a level of charge of the rechargeable battery. The biosignal monitoring system may further comprise a lead set removably coupled to the housing. The wireless signal acquisition device may include a lead set status indicator positioned on the housing and configured to illuminate to indicate connection of the lead set with the housing of the wireless signal acquisition device.

Optionally, the housing of the wireless signal acquisition device may define a front wall, a back wall opposite the front wall, and a sidewall extending between and interconnecting the front wall and the back wall. The back wall of the wireless signal acquisition device may engage a forwardly-facing surface of the first acquisition-device receiver while the wireless signal acquisition device is positioned in the first acquisition-device receiver of the dock. An entirety of the back wall of the wireless signal acquisition device may engage the dock while the wireless signal acquisition device is positioned in the first acquisition-device receiver of the dock and the front wall of the wireless signal acquisition device may be visible while the wireless signal acquisition device is positioned in the dock. The dock may include a front wall and a sidewall coupled to the front wall. The first acquisition-device receiver may comprise a recess that extends inwardly into the dock from the front wall. The recess may be defined by a forwardly-facing surface and a side surface extending between and interconnecting the forwardly-facing surface of the recess and the front wall of the dock.

Further optionally, the forwardly-facing surface of the recess may be parallel to the front wall of the housing of the wireless signal acquisition device while the wireless signal acquisition device is positioned in the first acquisition-device receiver of the dock. The dock may be formed to include a lead set groove extending outwardly from the recess to receive a portion of the wireless signal acquisition device therein while the wireless signal acquisition device is positioned in the first acquisition-device receiver of the dock. The dock may be formed to include a grip that extends outwardly from the recess to facilitate gripping of the housing of the wireless signal acquisition device to remove the wireless signal acquisition device from the dock. The grip may include a first grip groove and a second grip groove opposite the first grip groove. Each of the first grip groove and the second grip groove may extend outwardly from the recess.

If desired, the dock may be mountable to a wall in a stationary position. A forwardly-facing surface of the first acquisition-device receiver may be parallel to the wall while the dock is mounted to the wall. The dock may be configured to be supported on a horizontal surface while the dock is in use. The dock may be mountable to a pole and the dock may be stationary relative to the pole while the dock is mounted to the pole. The dock may be formed to include a second acquisition-device receiver spaced apart from the first acquisition-device receiver. The dock may be formed to include a third acquisition-device receiver spaced apart from the second acquisition-device receiver.

Further according to the present disclosure, a wireless signal acquisition device may be provided. The wireless signal acquisition device may be configured to acquire biosignals of a patient. The wireless signal acquisition device may be configured to wirelessly communicate with a dock. The wireless signal acquisition device may comprise a housing, a device charging unit, and a docking positioner. The housing may include a front wall, a back wall opposite the front wall, and a sidewall extending between and interconnecting the front wall and the back wall. The front wall, the back wall, and the sidewall may cooperate to form an electronics receiving-space. The device charging unit may be positioned in the electronics receiving-space of the housing. The device charging unit may include a rechargeable battery configured to provide power to the wireless signal acquisition device and a power receiver configured to recharge the rechargeable battery. The docking positioner may be coupled to the housing and may be configured to aid magnetic alignment and biasing of the wireless signal acquisition device within the dock so that the power receiver of the device charging unit of the wireless signal acquisition device is aligned with the dock for wireless recharging of the rechargeable battery.

Optionally, the docking positioner may comprise a ferromagnetic metal component. The detected biosignals may be electrocardiogram signals. The wireless signal acquisition device may further comprise a lead set including a first end removably coupled to the housing and a second end opposite the first end. The docking positioner and the lead set may be positioned at opposing ends of the wireless signal acquisition device. The wireless signal acquisition device may further comprise a lead set status indicator positioned on the housing. The lead set status indicator may be configured to illuminate to indicate connection of the first end of the lead set with the housing. The second end may provide a 12 lead diagnostic signal. The second end may provide a 15 lead diagnostic signal. The second end may provide a 5 lead diagnostic signal. The second end may provide a 3 lead diagnostic signal.

Further optionally, the wireless signal acquisition device may further comprise a first button positioned on the housing. The first button may be configured to be actuated to start an electrocardiogram exam. The wireless signal acquisition device may further comprise a biosignal status indicator extending around a perimeter of the first button and configured to illuminate to indicate acquisition of the biosignals during the electrocardiogram exam. The wireless signal acquisition device may further comprise a second button positioned on the housing. The second button may be configured to be actuated to start a rhythm exam. The wireless signal acquisition device may further comprise a biosignal status indicator extending around a perimeter of the second button and configured to illuminate to indicate acquisition of the biosignals during the rhythm exam.

If desired, the wireless signal acquisition device may further comprise a third button positioned on the housing. The third button may be configured to be actuated to power on, to power off, and to wake up the wireless signal acquisition device. The wireless signal acquisition device may further comprise a charge status indicator positioned on the housing. The charge status indicator may be configured to illuminate to indicate a level of charge of the rechargeable battery.

Further according to the present disclosure, a dock configured to engage wirelessly with a wireless signal acquisition device may be provided. The dock may comprise a body, a port, a charging unit, and a docking positioner. The body may be formed to include a first acquisition-device receiver to receive the wireless signal acquisition device therein. The port may extend into the body and may be configured to receive a power cable therein to provide power to the dock. The charging unit may be positioned inside the body of the dock. The docking positioner may be coupled to the body. The docking positioner may be configured to magnetically align the wireless signal acquisition device within the dock so that the charging unit of the dock is aligned with the wireless signal acquisition device for wireless recharging of the wireless signal acquisition device.

Alternatively, the docking positioner may comprise a static magnet and a steel shunt. The steel shunt may receive the static magnet therein so that a magnetic field of the static magnet is concentrated near an outwardly facing surface of the static magnet.. The body of the dock may include a front wall and a sidewall coupled to the front wall. The first acquisition-device receiver may comprise a recess that extends inwardly into the body from the front wall. The recess may be defined by a forwardly-facing surface and a side surface extending between and interconnecting the forwardly-facing surface of the recess and the front wall of the body. The dock may further comprise a power availability status indicator positioned on the forwardly-facing surface of the recess and configured to indicate a power availability of the dock.

Additionally, the body may be formed to include a lead set groove extending outwardly from the recess. The lead set groove and the docking positioner may be positioned on opposing ends of the dock. The body may be formed to include a grip that extends outwardly from the recess. The grip may include a first grip groove and a second grip groove opposite the first grip groove. Each of the first grip groove and the second grip groove may extend outwardly from the recess.

Optionally, the dock may be mountable to a wall. The forwardly-facing surface of the first acquisition-device receiver may be parallel to the wall while the dock is mounted to the wall. The dock may be configured to be supported on a horizontal surface while the dock is in use. The dock may be mountable to a pole and the dock may be stationary relative to the pole while the dock is mounted to the pole. The body of the dock may include a front wall, a sleeve wall in spaced apart relation to the front wall, and a sidewall extending outwardly from the front wall and interconnecting the front wall and the sleeve wall. The front wall, the sidewall, and the sleeve wall may cooperate to provide the first acquisition-device receiver.

Further optionally, the dock may be formed to include a lead set holder configured to extend around at least a portion of the wireless signal acquisition device to maintain a position of the wireless signal acquisition device relative to the dock while the wireless signal acquisition device is positioned in the dock. The body may be formed to include a lead set groove extending outwardly from the first acquisition-device receiver. The body may be formed to include a second acquisition-device receiver spaced apart from the first acquisition-device receiver. The body may be formed to include a third acquisition-device receiver spaced apart from the second acquisition-device receiver. The dock may be integrated with a cardiograph.

Further according to the present disclosure, a biosignal monitoring system may be provided. The biosignal monitoring system may comprise a wireless signal acquisition device and a dock. The wireless signal acquisition device may be configured to acquire biosignals of a patient. The wireless signal acquisition device may include a device charging unit positioned inside of the wireless signal acquisition device. The device charging unit may include a rechargeable battery configured to provide power to the wireless signal acquisition device and a power receiver in electrical communication with the rechargeable battery. The power receiver may have a receiver coil configured to recharge the rechargeable battery. The receiver coil may be defined by a plurality of spiral layers that each extend circumferentially around a central axis of the receiver coil. Each of the plurality of spiral layers may be equidistant from the central axis of the receiver coil. The dock may be configured to receive the wireless signal acquisition device therein and wirelessly engage with the wireless signal acquisition device. The dock may include a dock charging unit positioned inside of the dock. The dock charging unit may include a transmitter coil. The transmitter coil may be defined by a plurality of spiral layers that each extend circumferentially around a central axis of the transmitter coil. Each of the plurality of spiral layers of the transmitter coil may be equidistant from the central axis of the transmitter coil. In response to the receiver coil being concentrically aligned with the transmitter coil while the wireless signal acquisition device is positioned in the dock, an electric current may be induced in the receiver coil due to electromagnetic induction so that the rechargeable battery is wirelessly recharged via inductive resonant charging.

Optionally, the wireless signal acquisition device may include a device communication module having a wireless data transmitter and a wireless data receiver. The wireless data receiver may be configured to acquire the biosignals and communicate the biosignals to the wireless data transmitter. The wireless data transmitter may be configured to wirelessly transmit the biosignals to the dock.

Further optionally, the dock may include a dock communication module configured to wirelessly receive the biosignals from the wireless data transmitter of the device communication module. The wireless data transmitter of the device communication module may be configured to wirelessly transmit power feedback data to the dock communication module indicative of a power level of the rechargeable battery.

If desired, the wireless signal acquisition device may include a device pairing module positioned inside of the wireless signal acquisition device. The dock may include a dock pairing module positioned inside of the dock. The device pairing module and the dock pairing module may communicate with one another to form a wireless link between the device communication module and the dock communication module for wireless communication therebetween. The device pairing module and the dock pairing module may automatically communicate with one another to form the wireless link in response to the wireless signal acquisition device being positioned in the dock.

Alternatively, the device pairing module may act as an active near-field communication tag and the dock pairing module may act as a near-field communication reader. The device pairing module may include a near-field communication tag coil and the dock pairing module may include a near-field communication reader coil. The near-field communication tag coil may be fabricated as part of a first printed circuit board assembly of the wireless signal acquisition device and the near-field communication reader coil may be fabricated as part of a second printed circuit board assembly of the dock. Wireless recharging of the rechargeable battery may automatically occur in response to the wireless signal acquisition device being placed in the dock.

Additionally, the dock may be coupled to a host device to receive power therefrom and to provide power to the dock charging unit. Each of the plurality of spiral layers of the receiver coil may be made of a flat copper sheet. The receiver coil may include a plurality of vias that extend between and interconnect each of the plurality of spiral layers of the receiver coil to transfer signals between each of the plurality of spiral layers of the receiver coil. Each of the plurality of spiral layers of the transmitter coil may be made of a flat copper sheet. The transmitter coil may include a plurality of vias that extend between and interconnect each of the plurality of spiral layers of the transmitter coil to transfer signals between each of the plurality of spiral layers of the transmitter coil. The wireless signal acquisition device may include a ferrite plate arranged between the rechargeable battery and the receiver coil.

Further according to the present disclosure, a control system for a biosignal monitoring system is provided. The control system includes a device communication module, a dock communication module, and a pairing module assembly. The device communication module may be arranged in a wireless signal acquisition device of the biosignal monitoring system and configured to acquire biosignals of a patient. The device communication module may include a wireless data transmitter configured to wirelessly communicate the biosignals to a dock and a wireless data receiver configured to acquire the biosignals and communicate the biosignals to the wireless data transmitter. The dock communication module may be arranged in the dock of the biosignal monitoring system and configured to wirelessly receive the biosignals from the wireless data transmitter of the device communication module. The pairing module assembly may be configured to securely pair the device communication module and the dock communication module to form a wireless link for wireless communication therebetween. The pairing module assembly may include a device pairing module arranged in the wireless signal acquisition device and a dock pairing module arranged in the dock. The wireless link may be automatically formed in response to the wireless signal acquisition device being positioned in the dock.

Optionally, the control system may further comprise a device charging unit positioned inside of the wireless signal acquisition device to power the wireless signal acquisition device and a dock charging unit positioned inside of the dock to wirelessly recharge the device charging unit while the wireless signal acquisition device is positioned in the dock. The device charging unit may include a rechargeable battery configured to provide power to the wireless signal acquisition device and a power receiver in electrical communication with the rechargeable battery. The power receiver may have a receiver coil configured to recharge the rechargeable battery. The wireless data transmitter of the device communication module of the wireless signal acquisition device may be configured to wirelessly communicate power feedback data to the dock communication module indicative of a power level of the rechargeable battery.

Further optionally, the receiver coil may be defined by a plurality of spiral layers that each extend circumferentially around a central axis of the receiver coil. Each of the plurality of spiral layers may be equidistant from the central axis of the receiver coil. Each of the plurality of spiral layers of the receiver coil may be made of a flat copper sheet. The dock charging unit may include a transmitter coil defined by a plurality of spiral layers that each extend circumferentially around a central axis of the transmitter coil. Each of the plurality of spiral layers of the transmitter coil may be equidistant from the central axis of the transmitter coil. In response to the receiver coil being aligned with the transmitter coil while the wireless signal acquisition device is positioned in the dock, an electric current may be induced in the receiver coil due to electromagnetic induction so that the rechargeable battery is wirelessly recharged via inductive resonant charging.

If desired, the plurality of spiral layers of the transmitter coil may be made of a flat copper sheet. The dock may be coupled to a host device to receive power therefrom and to provide power to the dock charging unit. The dock communication module may communicate the biosignals to the host device. The device pairing module may act as a near-field communication tag and the dock pairing module may act as a near-field communication reader. The device pairing module may include a near-field communication tag coil and the dock pairing module may include a near-field communication reader coil. The near-field communication tag coil may be fabricated as part of a first printed circuit board assembly of the wireless signal acquisition device and the near-field communication reader coil may be fabricated as part of a second printed circuit board assembly of the dock.

Further according to the present disclosure, a printed circuit board coil for use in inductive resonant charging of a wireless biosignal acquisition device is provided. The printed circuit board coil may include a plurality of spiral layers through which an alternating current flows, a plurality of vias, and a rectifier. The plurality of spiral layers may each extend circumferentially around a central axis of the printed circuit board coil. Each of the plurality of spiral layers may be axially spaced apart from one another. The plurality of vias may extend between and interconnect each of the plurality of spiral layers to transfer signals between each of the plurality of spiral layers. The rectifier may be configured to convert the alternating current into a direct current. Each spiral layer of the plurality of spiral layers may have an outer diameter that is the same for each spiral layer to cause the alternating current flowing through the plurality of spiral layers to be evenly distributed throughout each spiral layer of the plurality of spiral layers.

Optionally, each spiral layer of the plurality of spiral layers may be made of a flat copper sheet. The plurality of spiral layers may include at least four layers.

Further according to the present disclosure, a biosignal monitoring system is provided. The biosignal monitoring system includes a wireless signal acquisition device and a dock. The wireless signal acquisition device may be configured to acquire biosignals of a patient and may include a device pairing module positioned inside of the wireless signal acquisition device. The dock may be configured to receive the wireless signal acquisition device therein and wirelessly engage with the wireless signal acquisition device. The dock may include a dock pairing module positioned inside of the dock and a printed circuit board assembly. The dock pairing module may be configured to pair with the device pairing module to establish a wireless link between the wireless signal acquisition device and the dock for wireless communication therebetween. The printed circuit board assembly may include a first capacitive plate electrode, a second capacitive plate electrode, and a capacitance monitoring circuit configured to detect a capacitance between the first capacitive plate electrode and the second capacitive plate electrode. In response to the wireless signal acquisition device being positioned on the dock, the capacitance monitoring circuit may detect a change in the capacitance between the first capacitive plate electrode and the second capacitive plate electrode. In response to the change in the capacitance, the capacitance monitoring circuit may initiate pairing between the device pairing module and the dock pairing module to establish the wireless link.

Optionally, the wireless signal acquisition device may include a device charging unit positioned inside of the wireless signal acquisition device. The device charging unit may include a rechargeable battery configured to provide power to the wireless signal acquisition device and a power receiver having a receiver coil configured to recharge the rechargeable battery. The receiver coil may be defined by a plurality of spiral layers that each extend circumferentially around a central axis of the receiver coil. Each of the plurality of spiral layers may be equidistant from the central axis of the receiver coil.

Further optionally, the dock may include a dock charging unit positioned inside of the dock and including a transmitter coil. The transmitter coil may be defined by a plurality of spiral layers that each extend circumferentially around a central axis of the transmitter coil. Each of the plurality of spiral layers of the transmitter coil may be equidistant from the central axis of the transmitter coil. In response to the change in the capacitance, the transmitter coil of the dock charging unit may automatically provide an initial minimum power amount to the receiver coil of the device charging unit.

If desired, the device pairing module may act as a near-field communication tag and the dock pairing module may act as a near-field communication reader. The device pairing module may include a near-field communication tag coil and the dock pairing module may include a near-field communication reader coil. The near-field communication reader coil may be fabricated as part of a printed circuit board of the printed circuit board assembly of the dock.

Further according to the present disclosure, a wireless signal acquisition device is provided. The wireless signal acquisition device includes a housing, an electric circuit, and a plurality of wire leads. The electric circuit may be carried by the housing. The plurality of wire leads may be coupled to the electric circuit and may extend from the housing. The electric circuit may include a rechargeable battery that is recharged via inductive resonant recharging, a communication module that wirelessly transmits acquired ECG data according to a wireless communication protocol, and a pairing module that wirelessly pairs the communication module with an external device according to a wireless pairing protocol.

Alternatively, the communication module may comprise a Bluetooth Low Energy (BLE) transmitter. The wireless communication protocol may be a BLE protocol. The pairing module may comprise a near field communication (NFC) chip. The wireless pairing protocol may be an NFC protocol.

The invention will now be further described by way of example with reference to the accompanying drawings, in which:

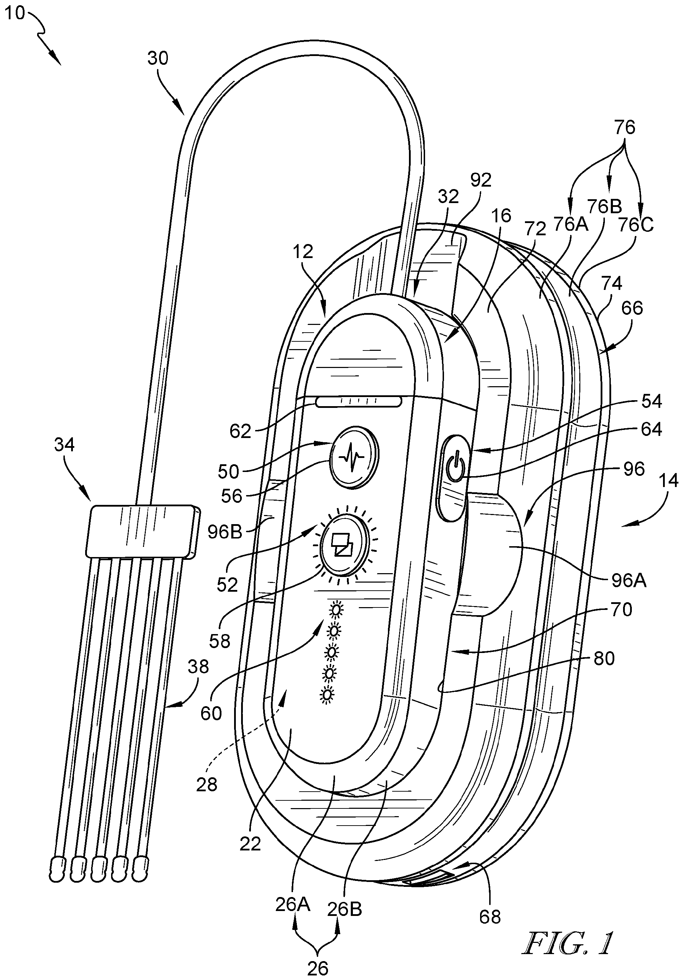

Fig. 1 is a perspective view of a biosignal monitoring system including a wireless signal acquisition device configured to acquire biosignals and a dock that is compatible with the wireless signal acquisition device such that the wireless signal acquisition device and the dock wirelessly communicate with one another and the dock wirelessly recharges a rechargeable battery of the wireless signal acquisition device while the wireless signal acquisition device is positioned on the dock;

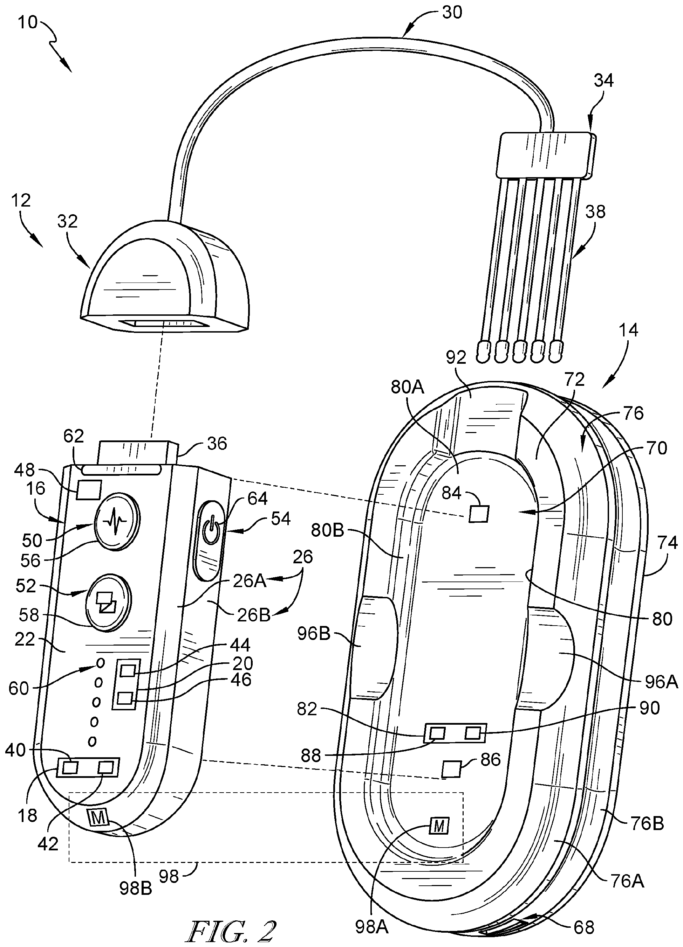

Fig. 2 is an exploded perspective view of the biosignal monitoring system of Fig. 1 showing that the dock is formed to include a first acquisition-device receiver to receive the wireless signal acquisition device therein for recharging of the rechargeable battery and further showing that the biosignal monitoring system includes a positioning assembly configured to position and align the wireless signal acquisition device relative to the dock so that the wireless recharging occurs, the positioning assembly including a magnet positioned on the dock and a ferromagnetic metal component positioned on the wireless signal acquisition device;

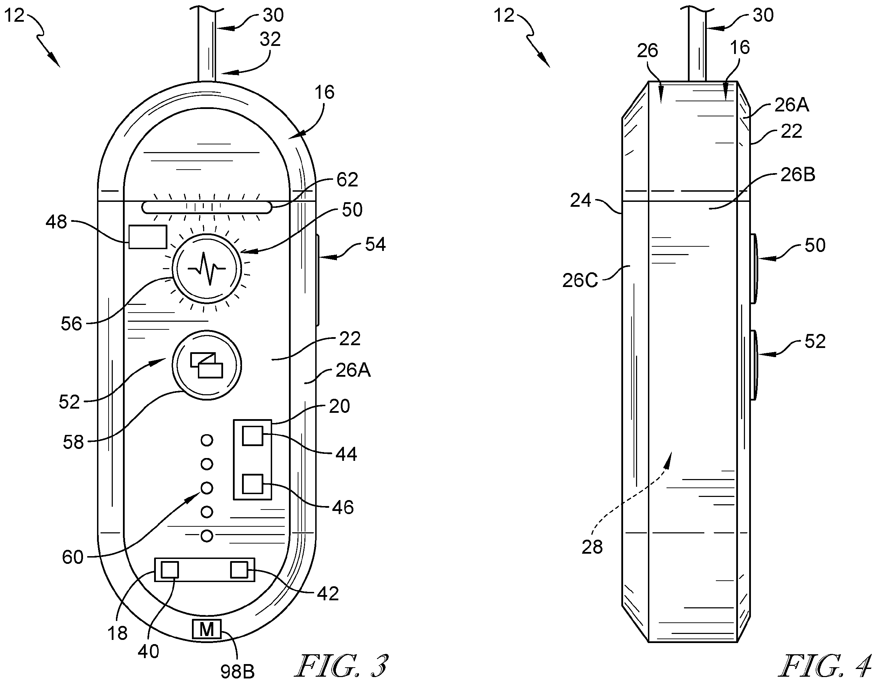

Fig. 3 is a front view of the wireless signal acquisition device of Fig. 1 showing that the wireless signal acquisition device includes a housing, a lead set removably coupled to the housing, a first button positioned on a front wall of the housing to control acquisition of the biosignals, a second button positioned on the front wall of the housing below the first button, a third button positioned on a sidewall of the housing to control on and off operation of the wireless signal acquisition device, a lead set status indicator positioned on the front wall of the housing above the first button to illuminate to indicate connection of the lead set with the wireless signal acquisition device, and a charge status indicator positioned on the front wall of the housing to illuminate to indicate a level of charge of the rechargeable battery;
Fig. 4 is a side view of the wireless signal acquisition device of Fig. 1 showing that the housing of the wireless signal acquisition device includes the front wall, a back wall opposite the front wall, and the sidewall extending between and interconnecting the front wall and the back wall, and further showing that the sidewall is defined by a first angled portion coupled to the front wall, a second portion coupled to the first angled portion, and a third angled portion interconnecting the second portion and the back wall;

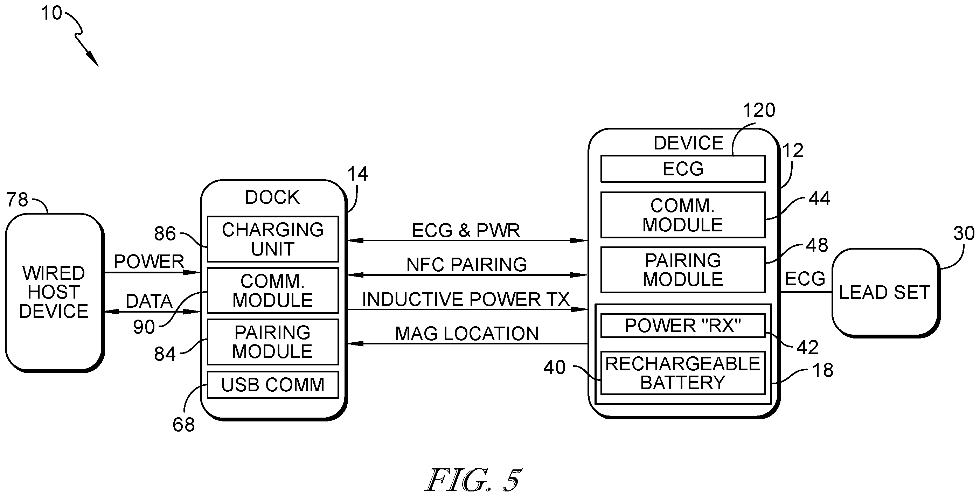

Fig. 5 is a diagrammatic view of the biosignal monitoring system of Fig. 1 showing that the wireless signal acquisition device and the dock wirelessly engage with one another to transmit biosignal data and power feedback data therebetween and to transmit power from the dock to the wireless signal acquisition device, and further showing that the dock is coupled with a host device to receive power therefrom and to transmit biosignal data thereto;

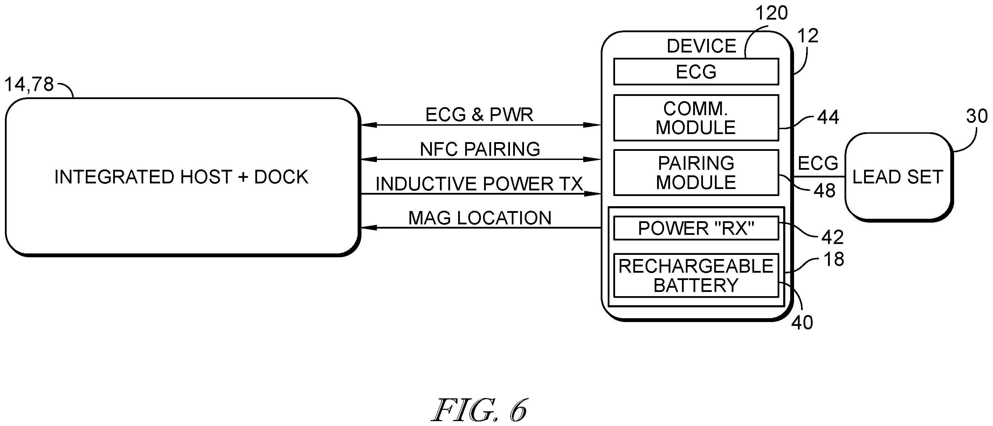

Fig. 6 is a diagrammatic view of a biosignal monitoring system including a dock that is integrated with a host device such that the wireless signal acquisition device of Fig. 3 wirelessly communicates with the integrated host and dock;

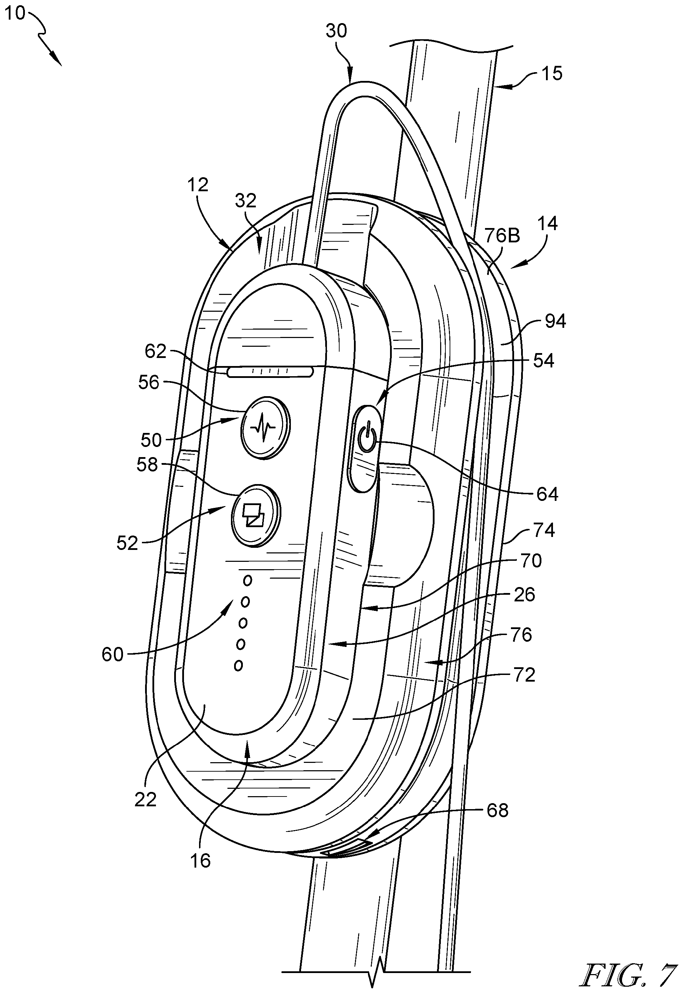

Fig. 7 is a perspective view of the biosignal monitoring system of Fig. 1 showing that the dock is mounted to a pole such that the dock is in a substantially vertical orientation and the wireless signal acquisition device is in a substantially vertical orientation while positioned in the first acquisition-device receiver of the dock, and further showing that the dock is formed to include a lead set holder to receive a portion of the lead set therein for storage of the lead set while the wireless signal acquisition device is positioned in the dock;

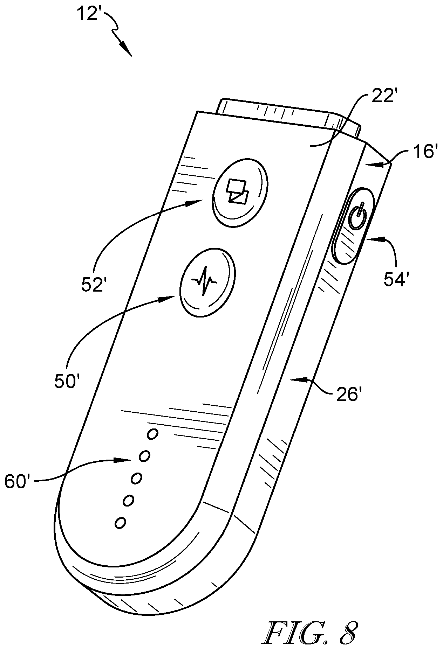

Fig. 8 is a perspective view of another embodiment of a wireless signal acquisition device for use with the dock of Fig. 1 showing that a lead set status indicator is omitted from the wireless signal acquisition device;

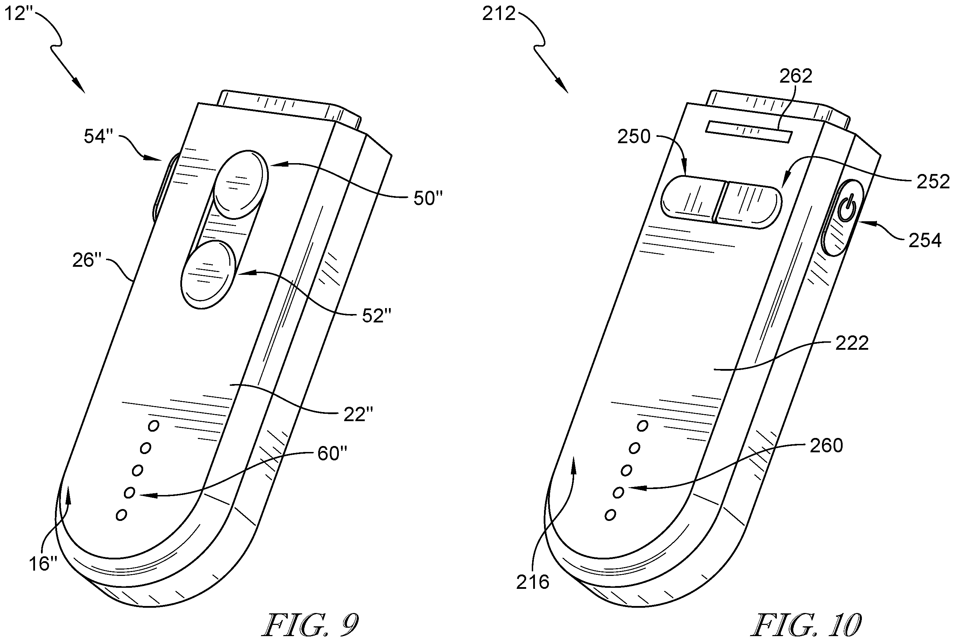

Fig. 9 is a perspective view of another embodiment of a wireless signal acquisition device for use with the dock of Fig. 1 showing that a third button is positioned on a sidewall of a housing opposite the positioning of the third button on the wireless signal acquisition device of Fig. 3;
Fig. 10 is a perspective view of another embodiment of a wireless signal acquisition device for use with the dock of Fig. 1 showing that a first button and a second button are positioned on a front wall of a housing side by side;

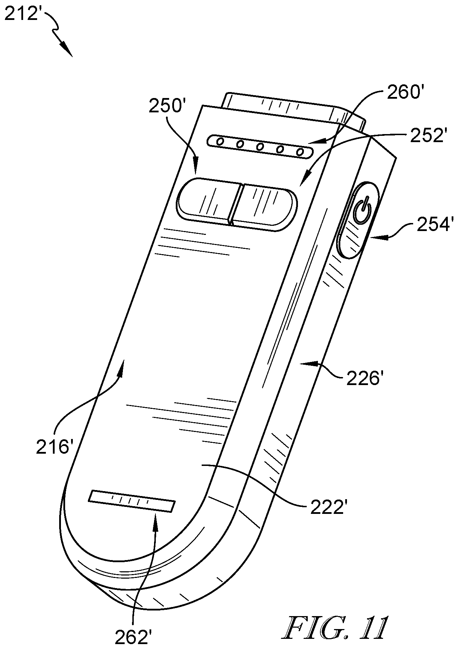

Fig. 11 is a perspective view of another embodiment of a wireless signal acquisition device for use with the dock of Fig. 1 showing that a first button and a second button are positioned on a front wall of a housing side by side, a lead set status indicator is positioned on the front wall of the housing below the first button and the second button, and a charge status indicator is positioned on the front wall of the housing above the first button and the second button;

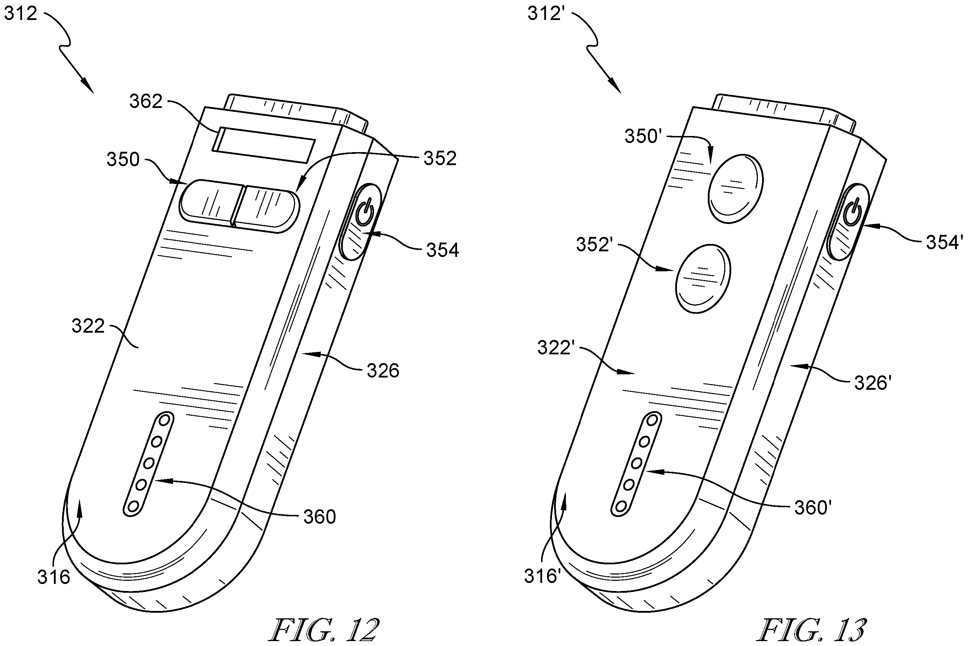

Fig. 12 is a perspective view of another embodiment of a wireless signal acquisition device for use with the dock of Fig. 1 showing that a first button and a second button are positioned on a front wall of a housing side by side, a lead set status indicator is positioned on the front wall of the housing above the first button and the second button, and a charge status indicator is positioned on the front wall of the housing below the first button and the second button;
Fig. 13 is a perspective view of another embodiment of a wireless signal acquisition device for use with the dock of Fig. 1 showing that a first button is positioned on a front wall of a housing, a second button is positioned on the front wall of the housing below the first button, and a charge status indicator is positioned on the front wall of the housing below the second button;

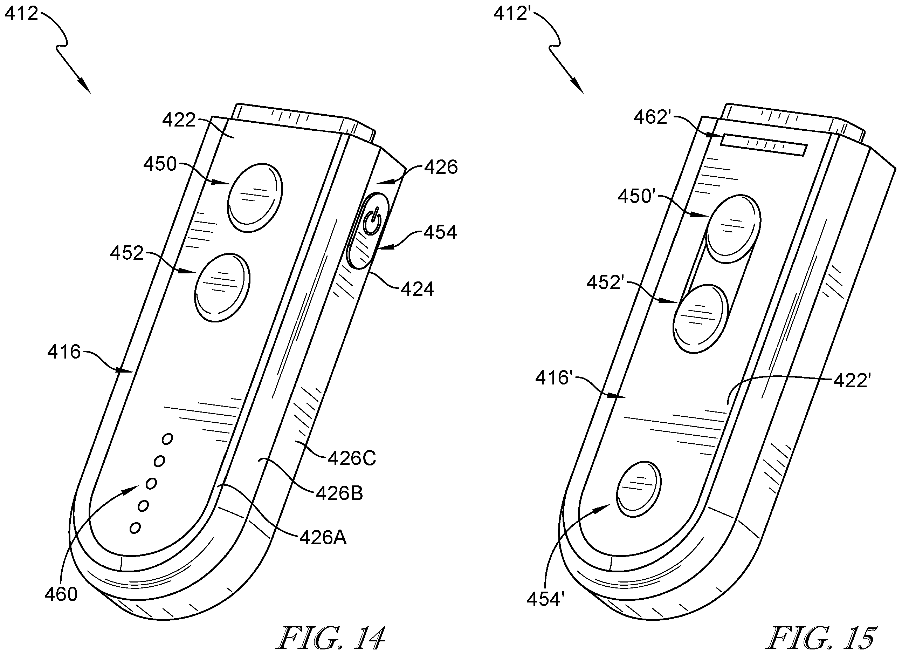

Fig. 14 is a perspective view of another embodiment of a wireless signal acquisition device for use with the dock of Fig. 1 showing that a front wall of a housing of the wireless signal acquisition device curves inwardly from a sidewall of the housing such that the front wall is recessed relative to the sidewall, and further showing that a first button is positioned on the front wall of the housing, a second button is positioned on the front wall of the housing below the first button, and a charge status indicator is positioned on the front wall of the housing below the second button;
Fig. 15 is a perspective view of another embodiment of a wireless signal acquisition device for use with the dock of Fig. 1 showing that a front wall of a housing of the wireless signal acquisition device curves inwardly from a sidewall of the housing such that the front wall is recessed relative to the sidewall, and further showing that a first button is positioned on the front wall of the housing, a second button is positioned on the front wall of the housing below the first button, a third button is positioned on the front wall of the housing below the second button, and a lead set status indicator is positioned on the front wall of the housing above the first button;

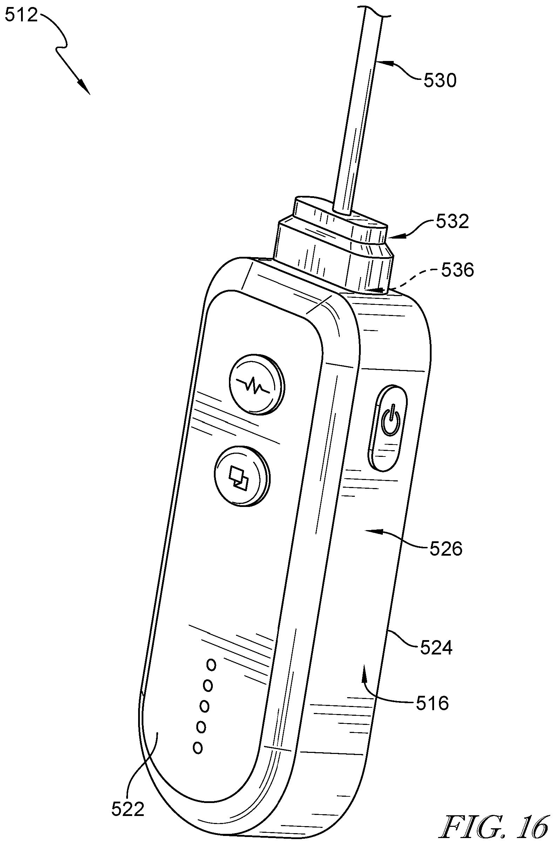

Fig. 16 is a perspective view of another embodiment of a wireless signal acquisition device for use with the dock of Fig. 1 showing that a lead set of the wireless signal acquisition device is configured to mate with a lead set connector formed in a housing of the wireless signal acquisition device;

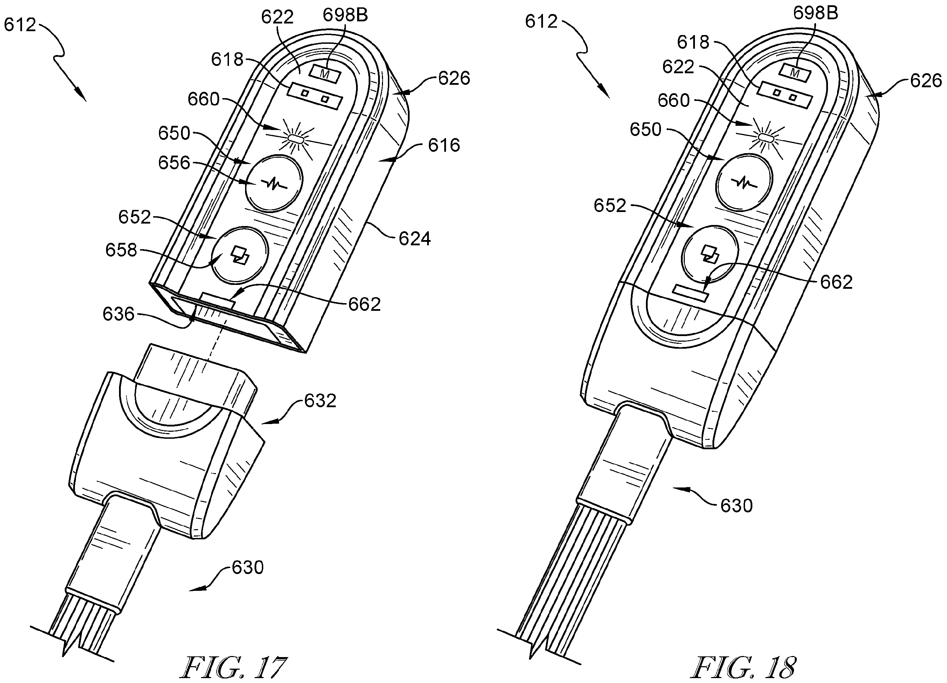

Fig. 17 is an exploded assembly view of another embodiment of a wireless signal acquisition device for use with the dock of Fig. 1 showing that a lead set of the wireless signal acquisition device is configured to mate with a lead set connector formed in a housing of the wireless signal acquisition device, and further showing that the wireless signal acquisition device includes a charge status indicator, a first button positioned on the housing below the charge status indicator, a second button positioned below the first button, and a lead set status indicator positioned on the housing below the second button;
Fig. 18 is a perspective view of the wireless signal acquisition device of Fig. 17 showing that a first end of the lead set forms a portion of the housing of the wireless signal acquisition device while the lead set is coupled with the housing, and further showing that a magnet of a positioning assembly is positioned on the wireless signal acquisition device at an end opposite the lead set connector;

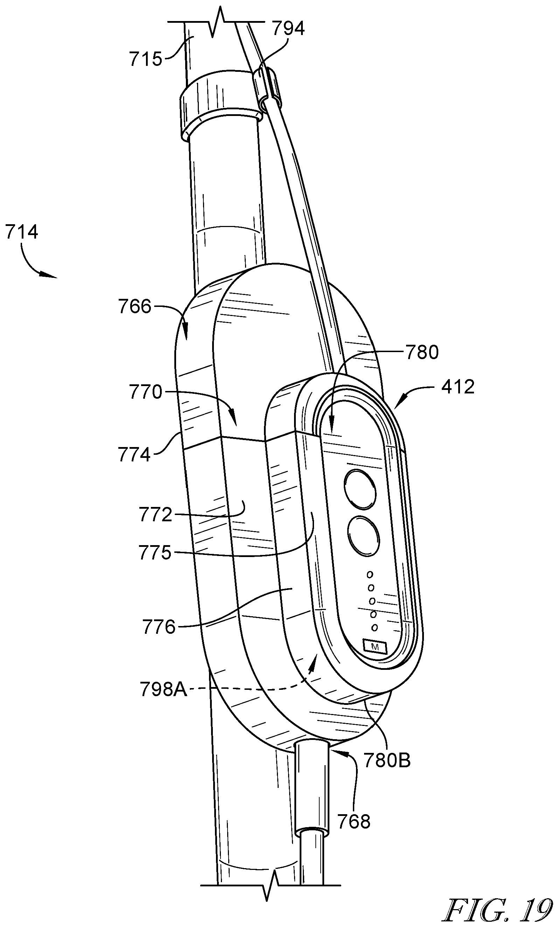

Fig. 19 is a perspective view of another embodiment of a dock mountable to a pole showing that the dock includes a body, a port extending inwardly into the body, and a first acquisition-device receiver formed as a sleeve to receive the wireless signal acquisition device of Figs. 3 and 8-18 therein;

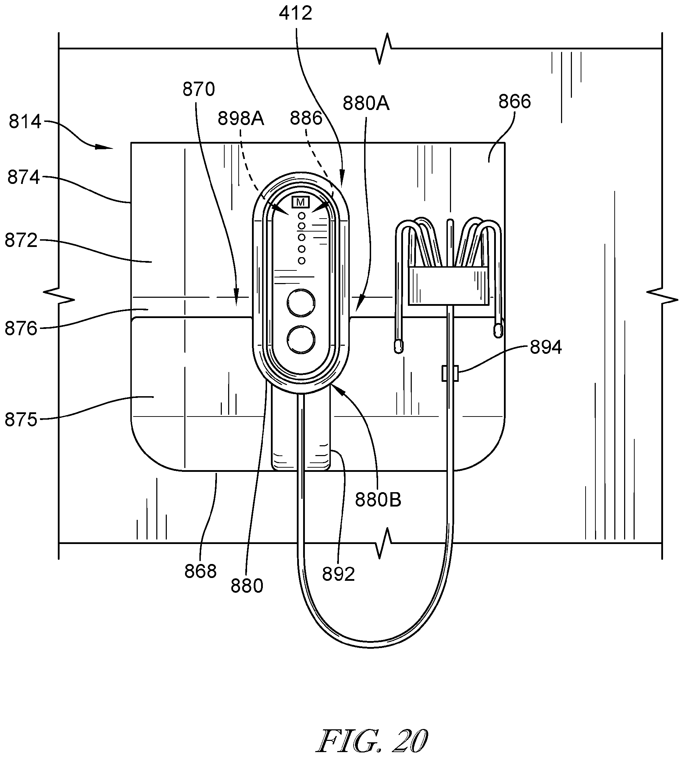

Fig. 20 is a perspective view of another embodiment of a dock mountable to a wall showing that the dock includes a body, a port extending inwardly into the body, and a first acquisition-device receiver formed as a pocket to receive the wireless signal acquisition device of Figs. 3 and 8-18 therein;

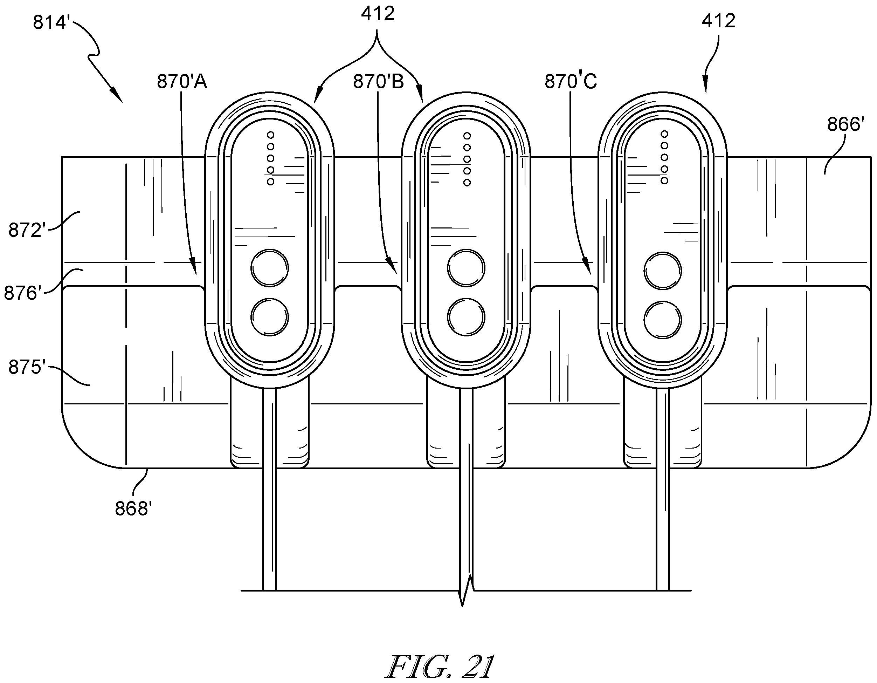

Fig. 21 is a perspective view of another embodiment of a dock mountable to a wall showing that the dock includes a body, a port extending inwardly into the body, a first acquisition-device receiver, a second acquisition-device receiver spaced apart from the first acquisition-device receiver, and a third acquisition-device receiver spaced apart from the second acquisition-device receiver, and further showing that each of the first acquisition-device receiver, the second acquisition-device receiver, and the third acquisition-device receiver is formed as a pocket to receive the wireless signal acquisition devices of Figs. 3 and 8-18 therein;

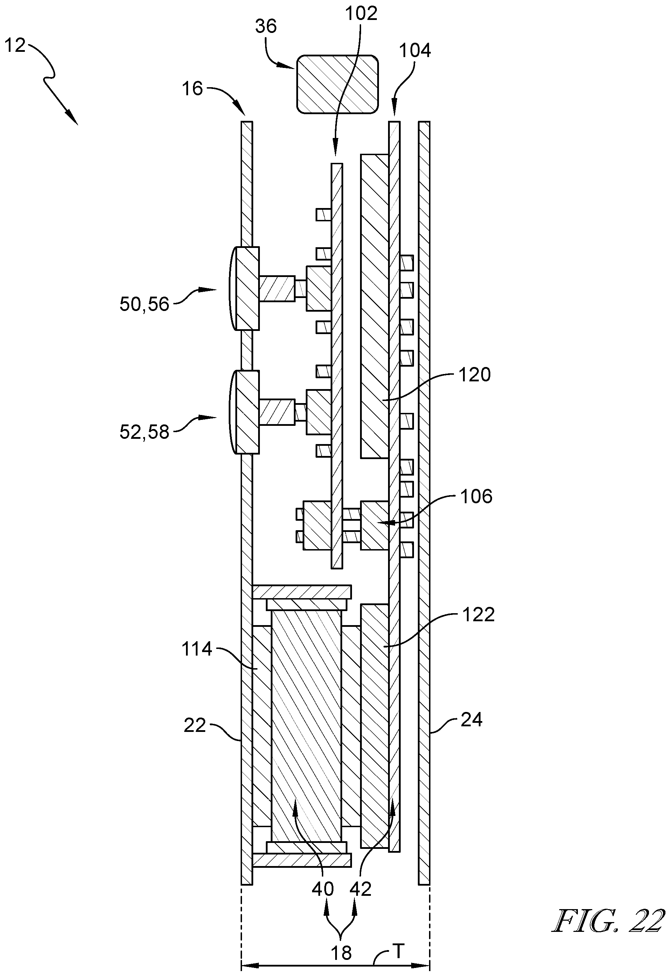

Fig. 22 is a diagrammatic cross-sectional view of the wireless signal acquisition device of Figs. 3 and 8-18 showing that the wireless signal acquisition device includes a first printed circuit board assembly and a second printed circuit board assembly, the first printed circuit board assembly arranged between the front wall of the housing of the wireless signal acquisition device and the second printed circuit board assembly, and the second printed circuit board assembly arranged between the first printed circuit board assembly and the back wall of the housing of the wireless signal acquisition device;

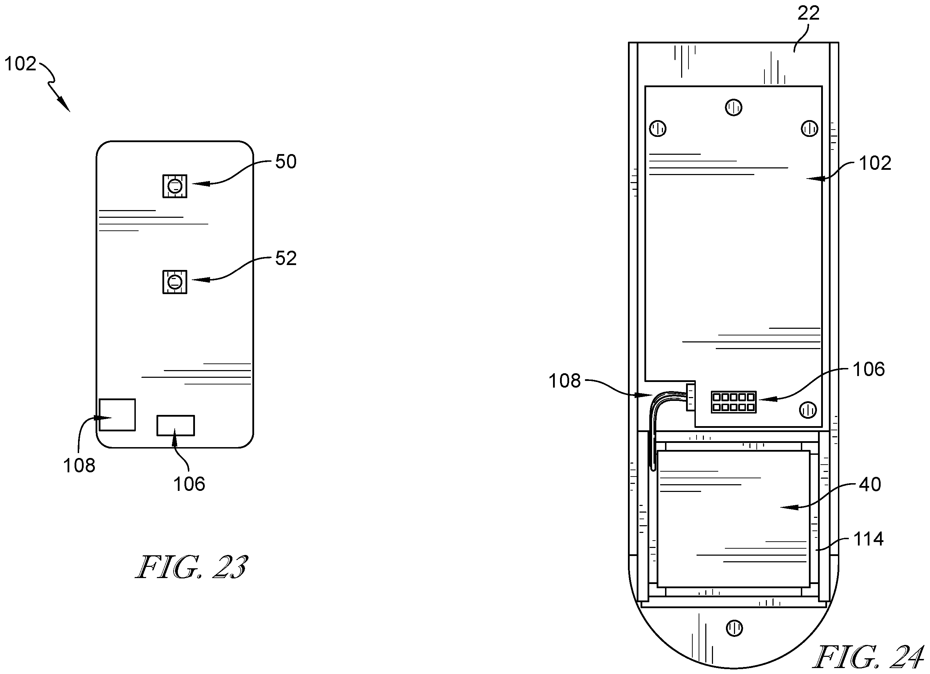

Fig. 23 is a diagrammatic front view of the first printed circuit board assembly of Fig. 22 showing that the first printed circuit board assembly interfaces with the first button and the second button of the wireless signal acquisition device;
Fig. 24 is a diagrammatic back view of the first printed circuit board assembly of Fig. 22 showing that the rechargeable battery is arranged below the first printed circuit board assembly and the rechargeable battery is coupled to the first printed circuit board assembly via a battery connector;

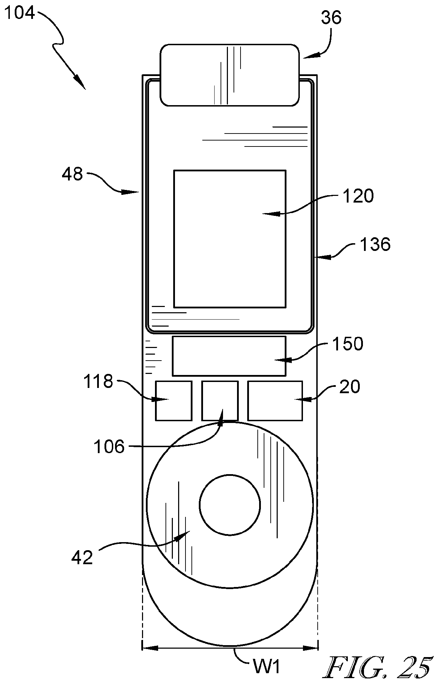

Fig. 25 is a diagrammatic front view of the second printed circuit board assembly of Fig. 22 showing that the second printed circuit board assembly includes an acquisition circuit thereon, a device pairing module including an NFC tag coil thereon, and a power receiver of a device charging unit thereon configured to charge the rechargeable battery, the power receiver including a receiver coil;

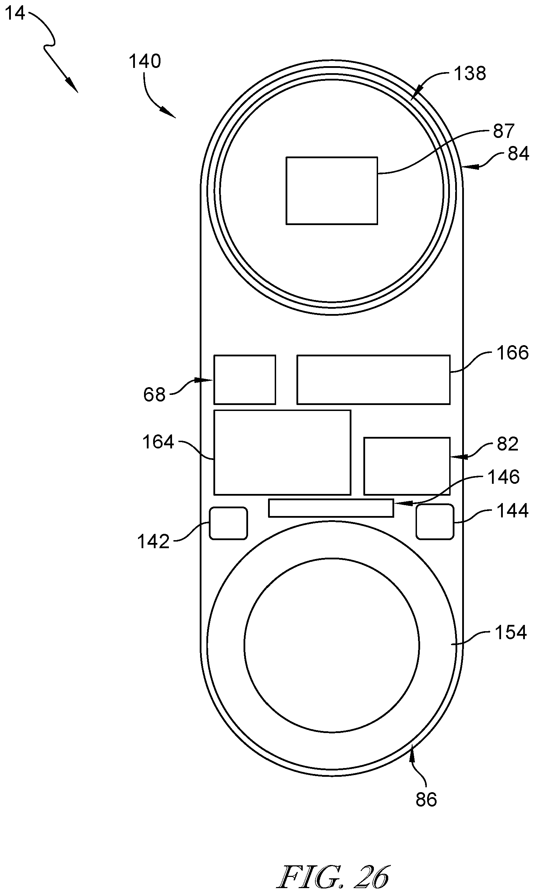

Fig. 26 is a diagrammatic view of a third printed circuit board assembly of the dock of Figs. 1-7 and 19-21 showing that the third printed circuit board assembly includes a dock pairing module thereon including an NFC reader coil that pairs with the device pairing module and a dock charging unit thereon including a transmitter coil configured to align with the receiver coil of Fig. 25 while the wireless signal acquisition device is positioned on the dock to charge the rechargeable battery via inductive resonant charging;

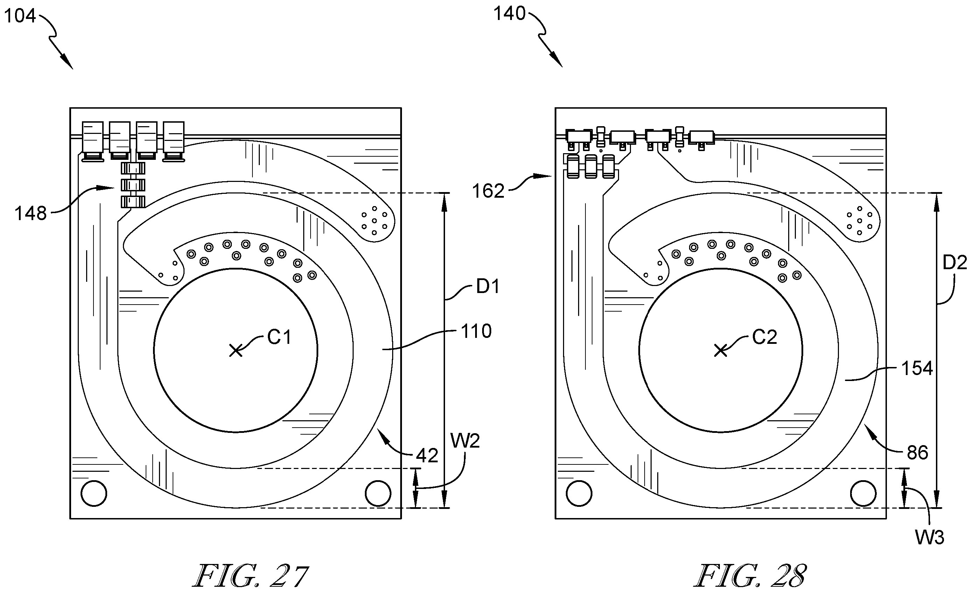

Fig. 27 is an enlarged view of a portion of the second printed circuit board assembly of Fig. 25 showing the receiver coil;
Fig. 28 is an enlarged view of a portion of the third printed circuit board assembly of Fig. 26 showing the transmitter coil and suggesting the transmitter coil and the receiver coil of Fig. 27 align with one another while the wireless signal acquisition device is positioned on the dock;

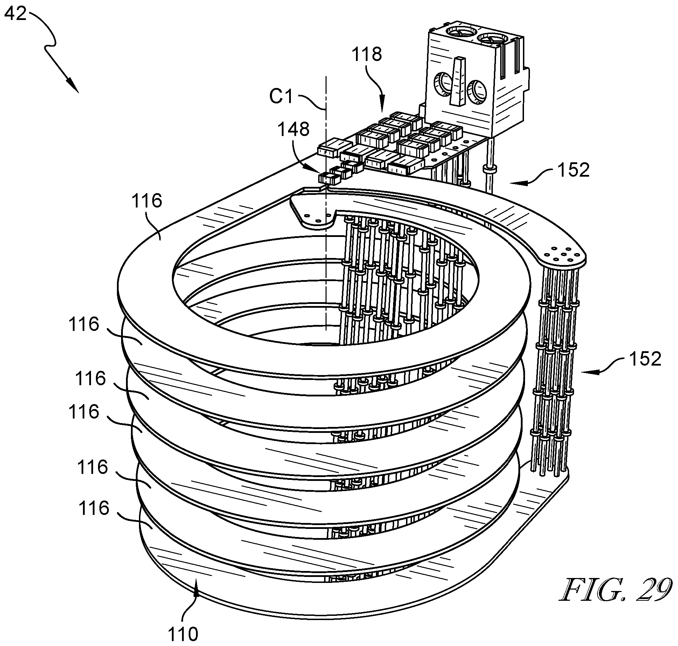

Fig. 29 is an expanded view of the receiver coil showing that the receiver coil includes a plurality of spiral layers that are each equidistant from a central axis of the receiver coil;

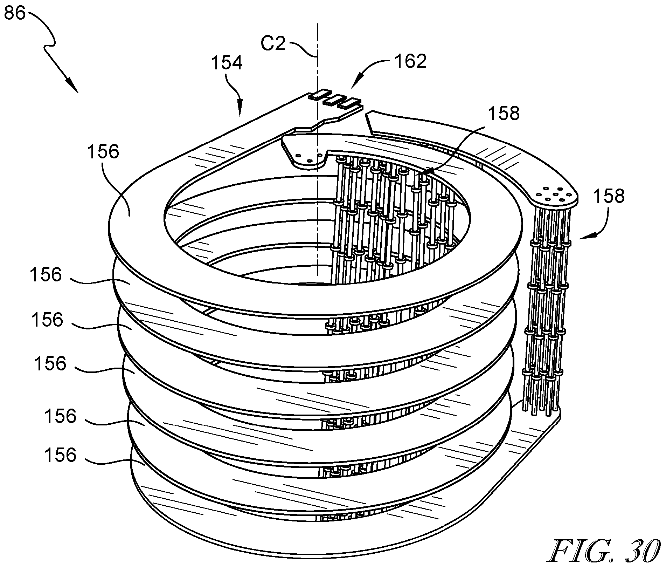

Fig. 30 is an expanded view of the transmitter coil showing that the transmitter coil includes a plurality of spiral layers that are each equidistant from a central axis of the transmitter coil;

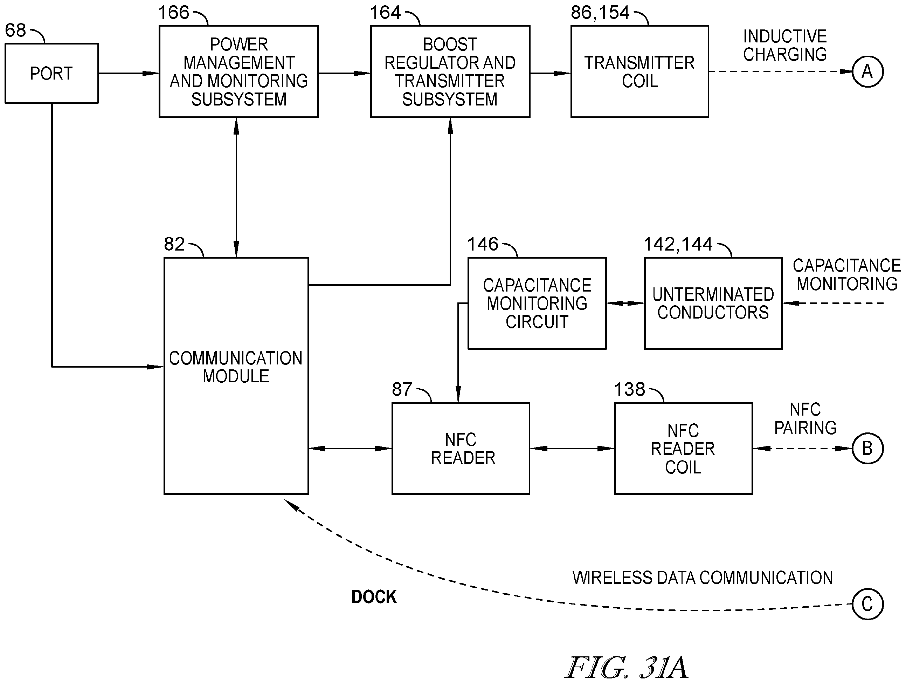

Fig. 31A is a first portion of a block diagram showing that the dock inductively charges the rechargeable battery of the wireless signal acquisition device via the transmitter coil, the NFC reader coil pairs with the NFC tag coil of the wireless signal acquisition device, and a dock communication module wirelessly communicates with the device communication module; and

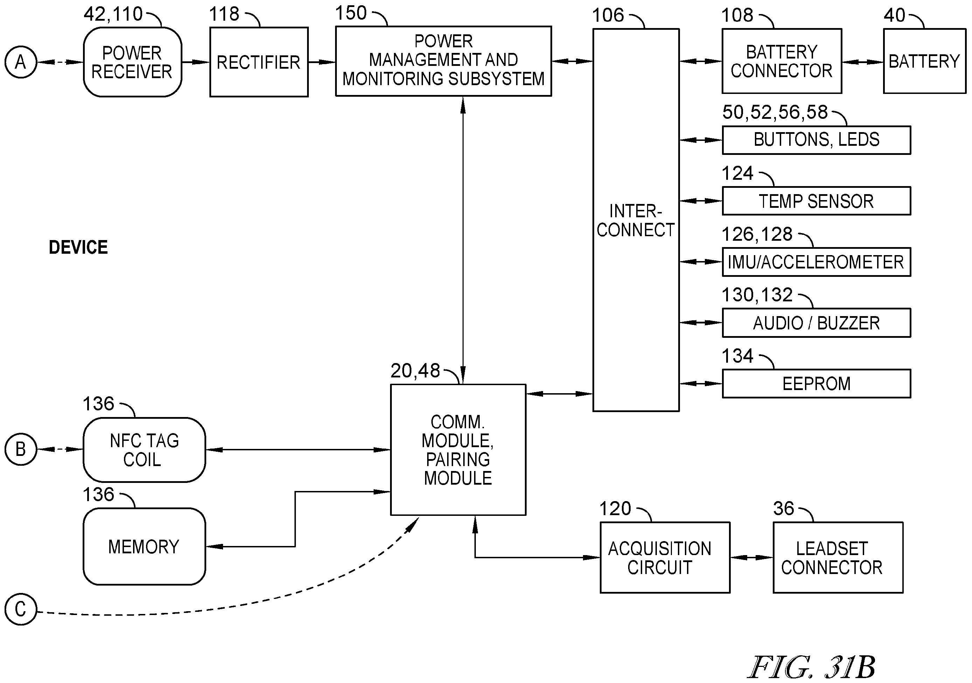

Fig. 31B is a second portion of the block diagram of Fig. 31A showing that the rechargeable battery is inductively charged via the transmitter coil, the NFC tag coil pairs with the NFC reader coil, and the device communication module wirelessly communicates with the dock communication module.

The present disclosure relates to a biosignal monitoring system 10. The biosignal monitoring system 10 includes a wireless signal acquisition device 12, a dock 14, and a positioning assembly 98 as shown in Figs. 1 and 2. The wireless signal acquisition device 12 is configured to acquire biosignals upon placement in contact with a patient. The wireless signal acquisition device 12 and the dock 14 are compatible with one another such that the wireless signal acquisition device 12 and the dock 14 wirelessly communicate with one another. The wireless signal acquisition device 12 is positioned in the dock 14 for wireless charging as shown in Fig. 1. The positioning assembly 98 aligns the wireless signal acquisition device 12 with the dock 14 for optimized charging. The wireless signal acquisition device 12 and the dock 14 engage wirelessly with one another to transmit data bidirectionally therebetween and to transmit power unidirectionally from the dock 14 to the wireless signal acquisition device 12.

Additional embodiments of wireless signal acquisition devices 12', 12", 212, 212', 312, 312', 412, 412', 512, 612 for use with the dock 14 are shown in Figs. 8-18. Additional embodiments of docks 714, 814, 814' for use with any of the wireless signal acquisition devices 12, 12', 12", 212, 212', 312, 312', 412, 412', 512, 612 are shown in Figs. 19-21.

The wireless signal acquisition device 12 includes a housing 16, a device charging unit 18, and a device communication module 20 as shown in Fig. 2. The housing 16 is illustratively formed as a clamshell enclosure that seals components of the wireless signal acquisition device 12 therein. The device charging unit 18 receives power from the dock 14 and provides power to the wireless signal acquisition device 12. The device communication module 20 transmits biosignal data and power feedback data to the dock 14.

In some embodiments, the detected biosignals by the wireless signal acquisition device 12 are electrocardiogram signals. In some embodiments, the detected biosignals by the wireless signal acquisition device 12 are photoplethysmogram signals. In some embodiments, the detected biosignals by the wireless signal acquisition device 12 are electroencephalogram signals. In some embodiments, the detected biosignals by the wireless signal acquisition device 12 are electromyography signals. In some embodiments, the detected biosignals by the wireless signal acquisition device 12 are respiratory signals indicative of respiratory rate. In some embodiments, the detected biosignals by the wireless signal acquisition device 12 are indicative of blood pressure. In some embodiments, the detected biosignals by the wireless signal acquisition device 12 are indicative of blood oxygen levels.

The housing 16 of the wireless signal acquisition device 12 is formed to include a front wall 22, a back wall 24 opposite the front wall 22, and a sidewall 26 extending between and interconnecting the front wall 22 and the back wall 24 as shown in Figs. 3 and 4. The front wall 22, the back wall 24, and the sidewall 26 cooperate to form an electronics receiving-space 28 within the housing 16. The device charging unit 18 and the device communication module 20 are received in the electronics receiving-space 28. In illustrative embodiments, the front wall 22 and the back wall 24 are substantially flat and parallel to one another.

The sidewall 26 is defined by a first angled portion 26A, a second portion 26B, and a third angled portion 26C as shown in Fig. 4. The first angled portion 26A extends between and interconnects the front wall 22 and the second portion 26B. The second portion 26B extends between and interconnects the first angled portion 26A and the third angled portion 26C. The third angled portion 26C extends between and interconnects the second portion 26B and the back wall 24. The second portion 26B is substantially perpendicular to the front wall 22 and the back wall 24. The first angled portion 26A and the third angled portion 26C of the sidewall 26 provide for an easier grip of the wireless signal acquisition device 12.

In some embodiments, the wireless signal acquisition device 12 includes a lead set 30 removably coupled to the housing 16 as shown in Figs. 1 and 2. The lead set 30 includes a first end 32 that interfaces with the wireless signal acquisition device 12 and a second end 34 opposite the first end 32. The first end 32 of the lead set 30 mates with a lead set connector 36 coupled with and extending away from the housing 16 of the wireless signal acquisition device 12 as suggested in Fig. 2. In some embodiments, an O-ring extends around the lead set connector 36 to prevent ingress of liquid into the electronics receiving-space 28. The second end 34 of the lead set 30 comprises a plurality of leads 38. Each of the plurality of leads 38 is configured to be attached to a patient to receive biosignals therefrom. In some embodiments, the plurality of leads 38 provides a 3 lead diagnostic signal. In some embodiments, the plurality of leads 38 provides a 5 lead diagnostic signal. In some embodiments, the plurality of leads 38 provides a 12 lead diagnostic signal. In some embodiments, the plurality of leads 38 provides a 15 lead diagnostic signal. The lead set connector 36 provides a standardized interface for lead sets 30 with a different number of leads 38. The wireless signal acquisition device 12 detects the number of leads 38 included in the lead set 30. Each lead 38 includes defibrillation protection resistors to allow for operation at low voltages.

As shown in Figs. 1 and 2, the first end 32 of the lead set 30 is shaped to match a shape of the housing 16 of the wireless signal acquisition device 12. While the first end 32 of the lead set 30 is coupled with the lead set connector 36, the lead set 30 and the housing 16 cooperate to define a continuous outer surface of the wireless signal acquisition device 12. The first end 32 of the lead set 30 provides a portion of the housing 16.

The device charging unit 18 of the wireless signal acquisition device 12 includes a rechargeable battery 40 and a power receiver 42 as shown in Fig. 2. The rechargeable battery 40 provides power to the wireless signal acquisition device 12 and is wirelessly recharged through inductive charging. The power receiver 42 receives current generated by the dock 14 to recharge the rechargeable battery 40. The rechargeable battery 40 and the power receiver 42 are arranged in the electronics receiving-space 28 of the housing 16 at an end of the wireless signal acquisition device 12 opposite the lead set connector 36.

The device communication module 20 of the wireless signal acquisition device 12 includes a wireless data transmitter 44 and a wireless data receiver 46 as shown in Fig. 2. The wireless data transmitter 44 and the wireless data receiver 46 wirelessly communicate with the dock 14. The wireless data transmitter 44 is configured to transmit biosignal data as detected by the lead set 30, power feedback data regarding the rechargeable battery 40, and/or service and sensor log data to the dock 14. The wireless data receiver 46 receives communication from the dock 14.

The wireless signal acquisition device 12 further includes a device pairing module 48 as shown in Fig. 2. Illustratively, the device pairing module 48 comprises a device near field communication ("NFC") chip 85 that enables the wireless signal acquisition device 12 to pair with the dock 14.

In some embodiments, the wireless signal acquisition device 12 further includes a first button 50, a second button 52, and/or a third button 54 as shown in Figs. 1 and 3. The first button 50, the second button 52, and the third button 54 are positioned on the housing 16. In some embodiments, the wireless signal acquisition device 12 further includes a first biosignal status indicator 56, a second biosignal status indicator 58, a charge status indicator 60, a lead set status indicator 62, and/or a power indicator 64. The first biosignal status indicator 56, the second biosignal status indicator 58, the charge status indicator 60, and the lead set status indicator 62 are positioned on the housing 16. In illustrative embodiments, the wireless signal acquisition device 12 is formed without a user interface screen to prevent ingress of liquid.

In some embodiments, the first button 50 and/or the second button 52 are coupled to the front wall 22 of the housing 16. In some embodiments, the first button 50 and/or the second button 52 are coupled to the sidewall 26 of the housing 16. In some embodiments, the first button 50 and the second button 52 have an Ingress Protection (IP) rating of 22. In some embodiments, the third button 54 is coupled to the second portion 26B of the sidewall 26. In some embodiments, the third button 54 is coupled to the front wall 22 of the housing 16. In some embodiments, the first biosignal status indicator 56, the second biosignal status indicator 58, the charge status indicator 60, and/or the lead set status indicator 62 are coupled to the front wall 22 of the housing 16. In some embodiments, the first biosignal status indicator 56, the second biosignal status indicator 58, the charge status indicator 60, and/or the lead set status indicator 62 are coupled to the sidewall 26 of the housing 16.

The first button 50 is configured to be actuated to control acquisition of the biosignals during various exams and/or to start various exams. For example, the first button 50 is pressed by the patient or a technician to start an electrocardiogram (ECG) exam. For example, the ECG exam may include acquiring ECG data for about ten seconds. In some embodiments, the first biosignal status indicator 56 extends around a perimeter of the first button 50 and is configured to illuminate to indicate that the ECG exam is ongoing as shown in Fig. 3. For example, the first biosignal status indicator 56 illuminates after the first button 50 is pressed and while the ECG exam is ongoing. In some embodiments, the first biosignal status indicator 56 comprises a light emitting diode.

In some embodiments, the second button 52 is configured to be actuated to control acquisition of the biosignals during various exams and/or to start various exams. For example, the second button 52 is pressed by the patient or the technician to start a rhythm exam. For example, the rhythm exam may include acquiring ECG data indefinitely or until the second button 52 is pressed again. For example, the second button 52 is pressed by the patient or the technician to start the rhythm exam. In some embodiments, the second biosignal status indicator 58 extends around a perimeter of the second button 52 and is configured to illuminate to indicate that the rhythm exam is ongoing as shown in Fig. 1. For example, the second biosignal status indicator 58 illuminates after the second button 52 is pressed and while the rhythm exam is ongoing. In some embodiments, the second biosignal status indicator 58 comprises a light emitting diode. In some embodiments, the second button 52 is positioned below the first button 50.

The third button 54 is configured to be actuated to power on, to power off, and to wake up the wireless signal acquisition device 12. In some embodiments, the power indicator 64 is positioned on the third button 54. In some embodiments, the power indicator 64 is positioned on the housing 16. The power indicator 64 is configured to illuminate to indicate actuation of the third button 54. For example, the third button 54 is pressed by the patient or the technician to turn the wireless signal acquisition device 12 on, and the power indicator 64 illuminates to indicate that the wireless signal acquisition device 12 is on. In some embodiments, the power indicator 64 comprises a light emitting diode.

The charge status indicator 60 is configured to illuminate to indicate a level of charge of the rechargeable battery 40 and/or whether recharging of the rechargeable battery 40 is actively ongoing. For example, the charge status indicator 60 may comprise five light emitting diodes, and if the rechargeable battery 40 is fully charged, all five of the light emitting diodes are illuminated as shown in Fig. 1. In some embodiments, the charge status indicator 60 may flash to indicate that the rechargeable battery 40 has a predetermined level of charge remaining. In some embodiments, the charge status indicator 60 may flash to indicate that the rechargeable battery 40 is recharging. The charge status indicator 60 is arranged below the second button 52 in a vertical orientation.

The lead set status indicator 62 is configured to illuminate to indicate connection of the first end 32 of the lead set 30 with the lead set connector 36 of the wireless signal acquisition device 12 as shown in Fig. 3. The lead set status indicator 62 is also configured to illuminate to indicate a status of the quality of the electrical connection to the patient (via the lead set 30). The lead set status indicator 62 is also configured to indicate the quality of the wireless connection with the dock 14 (i.e., whether the wireless connection has been established or has been lost). For example, the lead set status indicator 62 illuminates while the lead set 30 is properly connected to the lead set connector 36. As another example, the lead set status indicator 62 does not illuminate while the lead set 30 is not properly connected to the lead set connector 36. As another example, the lead set status indicator 62 illuminates in a first color, for example, green, to indicate that the lead set 30 is properly connected to the lead set connector 36 and the quality of the electrical connection to the patient is good. As another example, the lead set status indicator 62 illuminates in a second color, for example, yellow, to indicate that the lead set 30 is properly connected to the lead set connector 36 and the quality of the electrical connection to the patient is poor. As another example, the lead set status indicator 62 illuminates in the second color and flashes to indicate that the wireless signal acquisition device 12 needs service. As another example, the lead set status indicator 62 illuminates in a third color, for example, blue, and flashes to indicate that the wireless connection has been lost. The lead set status indicator 62 is arranged above the first button 50 adjacent the lead set connector 36 in a horizontal orientation. In some embodiments, the lead set status indicator 62 comprises a light emitting diode.

The dock 14 wirelessly communicates with the wireless signal acquisition device 12 and wirelessly charges the rechargeable battery 40 of the wireless signal acquisition device 12 as suggested in Fig. 5. The dock 14 includes a body 66, a port 68, and a first acquisition-device receiver 70 as shown in Figs. 1 and 2. The first acquisition-device receiver 70 is formed in the body 66 to receive the wireless signal acquisition device 12 therein. The port 68 extends into the body 66 and is configured to receive a power cable, such as a USB cable, therein to provide power to the dock 14. In some embodiments, the cable is internally connected with the dock 14. In other words, the cable is fixed to the dock 14 such that the cable extends out of the dock 14 and is not connected and disconnected from the dock 14.

The body 66 of the dock 14 is formed to include a front wall 72, a back wall 74 opposite the front wall 72, and a sidewall 76 extending between and interconnecting the front wall 72 and the back wall 74 as shown in Fig. 2. The sidewall 76 is defined by a first angled portion 76A, a second curved portion 76B, and a third angled portion 76C as shown in Fig. 2. The first angled portion 76A extends between and interconnects the front wall 72 and the second curved portion 76B. The second curved portion 76B extends between and interconnects the first angled portion 76A and the third angled portion 76C. The third angled portion 76C extends between and interconnects the second curved portion 76B and the back wall 74. The second curved portion 76B has a concave shape and curves inwardly as the second curved portion 76B extends from the first angled portion 76A to the third angled portion 76C.

The port 68 is illustratively formed as a USB 2.0 port that receives a power cable therein to connect the dock 14 to a host device 78 as shown in Fig. 5. The host device 78 may comprise a computer, a cardiograph, a stress system, or any other USB-featured display device. The dock 14 receives power from the host device 78 and transmits biosignal data to the host device 78 via the power cable. In some embodiments, the dock 14 and the host device 78 are integrated, as shown in Fig. 6, such that the wireless signal acquisition device 12 and the integrated dock and host device 14, 78 engage wirelessly with one another. For example, the dock 14 is integrated with a cart-mounted box cardiograph such that the wireless signal acquisition device 12 is positioned in the cart-mounted box cardiograph.

The first acquisition-device receiver 70 receives the wireless signal acquisition device 12 therein, as shown in Fig. 1, to charge the rechargeable battery 40 of the wireless signal acquisition device 12. The first acquisition-device receiver 70 comprises a recess 80 that extends inwardly into the body 66 from the front wall 72 of the body 66 toward the back wall 74 as shown in Fig. 2. The recess 80 is defined by a forwardly-facing surface 80A and a side surface 80B extending between and interconnecting the forwardly-facing surface 80A of the recess 80 and the front wall 72 of the body 66 of the dock 14. The forwardly-facing surface 80A of the recess 80 is parallel to the front wall 22 and the back wall 24 of the housing 16 of the wireless signal acquisition device 12 while the wireless signal acquisition device 12 is positioned in the dock 14.

In some embodiments, the dock 14 includes a power availability status indicator positioned on the forwardly-facing surface 80A of the recess 80. The power availability status indicator comprises an LED indicator. The power availability status indicator is configured to indicate to the user whether the dock 14 is ready to charge the wireless signal acquisition device 12. When the wireless signal acquisition device 12 is positioned on the dock 14, the power availability status indicator is not visible to the user as the power availability status indicator is located under the wireless signal acquisition device 12. The power availability status indicator is unobstructed from view when the wireless signal acquisition device 12 is not positioned on the dock 14, and the power availability status indicator is positioned in a clear line-of-sight location such that the user can determine whether the dock 14 is ready to charge the wireless signal acquisition device 12. However, while the wireless signal acquisition device 12 is docked, the power availability status indicator is obstructed and may be turned off via software. When docked, the wireless signal acquisition device 12 indicates the power status of the system 10.

While the wireless signal acquisition device 12 is positioned in the first acquisition-device receiver 70, the back wall 24 of the wireless signal acquisition device 12 engages the forwardly-facing surface 80A of the recess 80 as suggested in Fig. 1. In some embodiments, an entirety of the back wall 24 of the wireless signal acquisition device 12 engages the dock 14 while the wireless signal acquisition device 12 is positioned in the first acquisition-device receiver 70, and the front wall 22 of the wireless signal acquisition device 12 is visible while the wireless signal acquisition device 12 is positioned in the first acquisition-device receiver 70.

The dock 14 further includes a dock communication module 82, a dock pairing module 84, and a dock charging unit 86 as shown in Fig. 2. The dock communication module 82 communicates wirelessly with the device communication module 20 of the wireless signal acquisition device 12. The dock pairing module 84 enables pairing of the dock 14 with the device pairing module 48 of the wireless signal acquisition device 12 so that the dock 14 and the wireless signal acquisition device 12 can wirelessly engage with one another. The dock pairing module 84 illustratively comprises a dock NFC chip 87 that enables the dock 14 to pair with the wireless signal acquisition device 12. The dock charging unit 86 is configured to wirelessly recharge the rechargeable battery 40 of the device charging unit 18 of the wireless signal acquisition device 12 while the wireless signal acquisition device 12 is positioned in the first acquisition-device receiver 70.

The dock communication module 82 comprises a wireless data transmitter 88 and a wireless data receiver 90 as shown in Fig. 2. The wireless data transmitter 88 is configured to transmit power feedback data to the wireless data receiver 46 of the device communication module 20 of the wireless signal acquisition device 12. The wireless data transmitter 88 is configured to transmit firmware updates to the wireless data receiver 46 of the device communication module 20 of the wireless signal acquisition device 12. In some embodiments, the wireless data transmitter 88 is configured to transmit mode commands to the wireless data receiver 46 of the device communication module 20 of the wireless signal acquisition device 12, such as, for example, a mode command to start an ECG exam or a mode command to start a rhythm exam. The wireless data receiver 90 is configured to receive the biosignal data, the power feedback data, and/or the service and sensor log data from the wireless data transmitter 44 of the device communication module 20 of the wireless signal acquisition device 12.

The dock charging unit 86 cooperates with the power receiver 42 of the device charging unit 18 of the wireless signal acquisition device 12 to provide inductive charging to the rechargeable battery 40 of the wireless signal acquisition device 12 while the wireless signal acquisition device 12 is positioned in the recess 80 of the dock 14 as shown in Figs. 1 and 7. The dock charging unit 86 receives power from the host device 78 via the port 68. The dock charging unit 86 generates an electromagnetic field as electric current flows through the dock charging unit 86. While the wireless signal acquisition device 12 is positioned in the recess 80 of the dock 14, an electric current is induced in the power receiver 42 of the device charging unit 18 of the wireless signal acquisition device 12 due to electromagnetic induction. The electric current received by the power receiver 42 charges the rechargeable battery 40. Alignment of the power receiver 42 of the wireless signal acquisition device 12 with the dock charging unit 86 of the dock 14 allows for and/or maximizes recharging of the rechargeable battery 40. Thus, the dock charging unit 86 of the dock 14 is positioned on the dock 14 so that the dock charging unit 86 aligns with the power receiver 42 of the wireless signal acquisition device 12 while the wireless signal acquisition device 12 is positioned in the recess 80 of the dock 14.

The dock 14 is formed to include a lead set groove 92, a lead set holder 94, and a grip 96 as shown in Figs. 2 and 7. The lead set groove 92 is formed in the dock 14 and provides relief for a portion of the lead set 30 to pass through while the wireless signal acquisition device 12 is positioned on the dock 14. In some embodiments, the lead set holder 94 is formed in the body 66 of the dock 14 and is configured to extend around at least a portion of the lead set 30 while the wireless signal acquisition device 12 is received in the dock 14 to maintain a position of the lead set 30 relative to the dock 14. The grip 96 is formed in the dock 14 to facilitate removal of the wireless signal acquisition device 12 from the dock 14.

The lead set groove 92 extends outwardly from the recess 80 of the dock 14 as shown in Fig. 2. While the wireless signal acquisition device 12 is positioned in the dock 14, a portion of the lead set 30 adjacent the first end 32 of the lead set 30 passes through the lead set groove 92. The lead set groove 92 is formed in the body 66 at an end of the dock 14 opposite the dock charging unit 86. While the wireless signal acquisition device 12 is positioned on the dock 14, the dock charging unit 86 of the dock 14 is aligned concentrically with the device charging unit 18 of the wireless signal acquisition device 12, and the lead set 30 of the wireless signal acquisition device 12 is aligned with the lead set groove 92 of the body 66 of the dock 14.

In some embodiments, the lead set holder 94 is defined by the second curved portion 76B of the sidewall 76 of the dock 14 as shown in Fig. 7. For example, the lead set 30 passes through the lead set groove 92 and is received by the second curved portion 76B. A portion of the lead set 30 fits within the concave second curved portion 76B to secure the portion of the lead set 30 thereto. The lead set holder 94 provides lead set 30 management to reduce entanglement of the lead set 30.

The grip 96 extends outwardly from the recess 80 of the dock 14, as shown in Figs. 1 and 2, to facilitate gripping of the housing 16 of the wireless signal acquisition device 12 during removal the wireless signal acquisition device 12 from the dock 14. In some embodiments, the grip 96 includes a first grip groove 96A and a second grip groove 96B opposite the first grip groove 96A. Each of the first grip groove 96A and the second grip groove 96B extend outwardly from the recess 80 and are defined by concave portions of the front wall 72 of the body 66 of the dock 14. Fingers of the patient or the technician are received in the grip grooves 96A, 96B so that the patient or the technician can easily grip and remove the wireless signal acquisition device 12 from the recess 80 of the dock 14.

The positioning assembly 98 is configured to secure and align the wireless signal acquisition device 12 within the first acquisition-device receiver 70 (i.e., the recess 80) of the dock 14 to removably position the wireless signal acquisition device 12 in the first acquisition-device receiver 70. The positioning assembly 98 helps to align the power receiver 42 of the wireless signal acquisition device 12 with the dock charging unit 86 of the dock 14 so that the rechargeable battery 40 is wirelessly recharged.

In some embodiments, the positioning assembly 98 magnetically secures the wireless signal acquisition device 12 within the first acquisition-device receiver 70. The positioning assembly 98 comprises a magnet 98A, which may also be referred to as a docking positioner 98A, and a ferromagnetic metal component 98B, which may also be referred to as a docking positioner 98B. The magnet 98A is coupled to the body 66 of the dock 14. The ferromagnetic metal component 98B is coupled to the housing 16 of the wireless signal acquisition device 12.

In some embodiments, the magnet 98A is arranged inside of the body 66 of the dock 14. For example, the magnet 98A is arranged inside of the body 66 underneath the forwardly-facing surface 80A of the recess 80. The magnet 98A is located at an end of the dock 14 opposite the lead set groove 92. A pole of the magnet 98A faces outwardly toward the wireless signal acquisition device 12 so that, when the wireless signal acquisition device 12 is located near the dock 14, the ferromagnetic metal component 98B of the wireless signal acquisition device 12 is attracted to the magnetic field of the magnet 98A. The magnetic attraction helps to ensure that the wireless signal acquisition device 12 is correctly positioned on the dock 14 for charging and helps to keep the wireless signal acquisition device 12 in place during vibrations or movement (for example, if the device 12 and the dock 14 are being moved on a cart).

In some embodiments, the dock 14 includes a steel shunt that receives the magnet 98A therein. The steel shunt surrounds the magnet 98A entirely except for an outwardly facing surface of the magnet 98A that faces toward the wireless signal acquisition device 12. The steel shunt helps to concentrate the magnetic field of the magnet 98A toward the ferromagnetic metal component 98B of the wireless signal acquisition device 12, as opposed to having a spread-out magnetic field around the magnet 98A. As such, the wireless signal acquisition device 12 is more strongly held in place while positioned in the dock 14 and the attraction force between the magnet 98A and the ferromagnetic metal component 98B is more noticeable to the user as the user is positioning the wireless signal acquisition device 12 on the dock 14.

In some embodiments, the ferromagnetic metal component 98B is arranged inside of the housing 16 of the wireless signal acquisition device 12 (i.e., in the electronics receiving-space 28). For example, the ferromagnetic metal component 98B is arranged inside of the housing 16 near the dock charging unit 86. The ferromagnetic metal component 98B is located at an end of the wireless signal acquisition device 12 opposite the lead set 30.

In some embodiments, the magnet 98A is arranged outside of the body 66 of the dock 14. For example, the magnet 98A is coupled to an exterior of the body 66, such as the forwardly-facing surface 80A of the recess 80 or the side surface 80B of the recess 80.

In some embodiments, the ferromagnetic metal component 98B is arranged outside of the housing 16 of the wireless signal acquisition device 12. For example, the ferromagnetic metal component 98B is coupled to the back wall 24 of the housing 16 or the sidewall 26 of the housing 16.

In some embodiments, the positioning assembly 98 mechanically secures the wireless signal acquisition device 12 within the first acquisition-device receiver 70 of the dock 14. For example, a docking positioner, such as a tab, coupled to the wireless signal acquisition device 12 mates with a docking positioner, such as a receiver, coupled to the dock 14 to removably position the wireless signal acquisition device 12 in the first acquisition-device receiver 70.

The positioning assembly 98 also allows the dock 14 to be arranged in various orientations, for example, a horizontal orientation or a vertical orientation, while ensuring that the wireless signal acquisition device 12 is securely positioned within the dock 14 regardless of the orientation of the dock 14. In some embodiments, the dock 14 is configured to be supported on a horizontal surface while the dock 14 is in use. For example, the back wall 74 of the body 66 of the dock 14 is supported on the horizontal surface while the dock 14 is in use so that the recess 80 of the dock 14 is accessible and facing upwardly away from the horizontal surface. The dock 14 is in a horizontal orientation while the dock 14 is supported on the horizontal surface, and the wireless signal acquisition device 12 is in a horizontal orientation while the wireless signal acquisition device 12 is positioned in the dock 14.

In some embodiments, the dock 14 is mountable to a pole 15 as shown in Fig. 7. For example, the back wall 74 of the body 66 of the dock 14 is mountable to the pole 15 so that the dock 14 remains stationary relative to the pole 15. The dock 14 is in a vertical orientation while the dock 14 is mounted to the pole 15, and the wireless signal acquisition device 12 is in a vertical orientation while the wireless signal acquisition device 12 is positioned in the dock 14.

In some embodiments, the dock 14 is mountable to a wall in a stationary position. For example, the back wall 74 of the body 66 of the dock 14 is mountable to the wall so that the dock 14 remains stationary relative to the wall. The dock 14 is in a vertical orientation while the dock 14 is mounted to the wall. The forwardly-facing surface 80A of the recess 80 is parallel to the wall while the dock 14 is mounted to the wall.

Another embodiment of a wireless signal acquisition device 12' for use with the dock 14 is shown in Fig 8. The description of the wireless signal acquisition device 12 is incorporated by reference to apply to the wireless signal acquisition device 12', except in instances when it conflicts with the specific description and the drawings of the wireless signal acquisition device 12'. The wireless signal acquisition device 12' is similar to the wireless signal acquisition device 12 expect for different positioning of a first button 50' and a second button 52', and the lead set status indicator is omitted. The first button 50' is positioned on a front wall 22' of a housing 16' below the second button 52'. The second button 52' is positioned on the front wall 22' of the housing 16' above the first button 50'. A charge status indicator 60' is positioned on the front wall 22' of the housing 16' below the first button 50' in a vertical orientation. A third button 54' is coupled to a sidewall 26' of the housing 16' and is configured to be actuated to control an on and off operation of the wireless signal acquisition device 12'.

Another embodiment of a wireless signal acquisition device 12" for use with the dock 14 is shown in Fig 9. The description of the wireless signal acquisition device 12 is incorporated by reference to apply to the wireless signal acquisition device 12", except in instances when it conflicts with the specific description and the drawings of the wireless signal acquisition device 12". A first button 50" is positioned on a front wall 22" of a housing 16" above the second button 52". The second button 52" is positioned on the front wall 22" of the housing 16" below the first button 50". A charge status indicator 60" is positioned on the front wall 22" of the housing 16" below the second button 52" in a vertical orientation. The third button 54" is coupled to a sidewall 26" of the housing 16" and is configured to be actuated to control an on and off operation of the wireless signal acquisition device 12". As compared to the wireless signal acquisition device 12 shown in Fig. 3, the third button 54" is positioned on a left side of the wireless signal acquisition device 12", while the third button 54 of the wireless signal acquisition device 12 is positioned on a right side of the wireless signal acquisition device 12.

Another embodiment of a wireless signal acquisition device 212 for use with the dock 14 is shown in Fig 10. The description of the wireless signal acquisition device 12 is incorporated by reference to apply to the wireless signal acquisition device 212, except in instances when it conflicts with the specific description and the drawings of the wireless signal acquisition device 212. The wireless signal acquisition device 212 is similar to the wireless signal acquisition device 12 expect for different positioning of a first button 250 and a second button 252. The first button 250 is positioned on a front wall 222 of a housing 216 to a side of the second button 252. The second button 252 is positioned on the front wall 222 of the housing 216 to a side of the first button 250. A charge status indicator 260 is positioned on the front wall 222 of the housing 216 below each of the first button 250 and the second button 252 in a vertical orientation. A lead set status indicator 262 is positioned above each of the first button 250 and the second button 252 in a horizontal orientation. A third button 254 is coupled to a sidewall 226 of the housing 216 and is configured to be actuated to control an on and off operation of the wireless signal acquisition device 212.

Another embodiment of a wireless signal acquisition device 212' for use with the dock 14 is shown in Fig 11. The description of the wireless signal acquisition device 12 is incorporated by reference to apply to the wireless signal acquisition device 212', except in instances when it conflicts with the specific description and the drawings of the wireless signal acquisition device 212'. A first button 250' is positioned on a front wall 222' of a housing 216' to a side of a second button 252'. The second button 252' is positioned on the front wall 222' of the housing 216' to a side of the first button 250'. The charge status indicator 260' is positioned on the front wall 222' of the housing 216' above each of the first button 250' and the second button 252' in a horizontal orientation. The lead set status indicator 262' is positioned below each of the first button 250' and the second button 252' in a horizontal orientation. A third button 254' is coupled to a sidewall 226' of the housing 216' and is configured to be actuated to control an on and off operation of the wireless signal acquisition device 212'.

Another embodiment of a wireless signal acquisition device 312 for use with the dock 14 is shown in Fig 12. The description of the wireless signal acquisition device 12 is incorporated by reference to apply to the wireless signal acquisition device 312, except in instances when it conflicts with the specific description and the drawings of the wireless signal acquisition device 312. A first button 350 is positioned on a front wall 322 of a housing 316 to a side of a second button 352. The second button 352 is positioned on the front wall 322 of the housing 316 to a side of the first button 350. The charge status indicator 360 is positioned on the front wall 322 of the housing 316 below each of the first button 350 and the second button 352 in a vertical orientation. The lead set status indicator 362 is positioned above each of the first button 350 and the second button 352 in a horizontal orientation. A third button 354 is coupled to a sidewall 326 of the housing 316 and is configured to be actuated to control an on and off operation of the wireless signal acquisition device 312.

Another embodiment of a wireless signal acquisition device 312' for use with the dock 14 is shown in Fig 13. The description of the wireless signal acquisition device 12 is incorporated by reference to apply to the wireless signal acquisition device 312', except in instances when it conflicts with the specific description and the drawings of the wireless signal acquisition device 312'. A first button 350' is positioned on a front wall 322' of a housing 316' above a second button 352'. The second button 352' is positioned on the front wall 322' of the housing 316' below the first button 350'. The charge status indicator 360' is positioned on the front wall 322' of the housing 316' below the second button 352' in a vertical orientation. A third button 354' is coupled to a sidewall 326' of the housing 316' and is configured to be actuated to control an on and off operation of the wireless signal acquisition device 312'.

Another embodiment of a wireless signal acquisition device 412 for use with the dock 14 is shown in Fig 14. The description of the wireless signal acquisition device 12 is incorporated by reference to apply to the wireless signal acquisition device 412, except in instances when it conflicts with the specific description and the drawings of the wireless signal acquisition device 412. The wireless signal acquisition device 412 is similar to the wireless signal acquisition device 12 expect for a shape of a sidewall 426 of a housing 416 of the wireless signal acquisition device 412 and the omission of the lead set status indicator. The housing 416 is formed to include a front wall 422, a back wall 424 opposite the front wall 422, and a sidewall 426 extending between and interconnecting the front wall 422 and the back wall 424 as shown in Fig. 14.

The sidewall 426 is defined by a first curved portion 426A, a second angled portion 426B, and a third portion 426C as shown in Fig. 14. The first curved portion 426A extends between and interconnects the front wall 422 and the second angled portion 426B. The second angled portion 426B extends between and interconnects the first curved portion 426A and the third portion 426C. The third portion 426C extends between and interconnects the second angled portion 426B and the back wall 424. The third portion 426C is substantially perpendicular to the front wall 422. The first curved portion 426A curves inwardly from the second angled portion 426B to the front wall 422 such that the front wall 422 is positioned inwardly relative to the sidewall 426. The front wall 422 is recessed relative to the sidewall 426.

Each of a first button 450, a second button 452, and a charge status indicator 460 are positioned on the front wall 422 of the housing 416 as shown in Fig. 14. A third button 454 is coupled to the third portion 426C of the sidewall 426. The first button 450 is positioned above the second button 452. The charge status indicator 460 is arranged below the second button 452 in a vertical orientation.

Another embodiment of a wireless signal acquisition device 412' for use with the dock 14 is shown in Fig 15. The description of the wireless signal acquisition device 12 is incorporated by reference to apply to the wireless signal acquisition device 412', except in instances when it conflicts with the specific description and the drawings of the wireless signal acquisition device 412'. The wireless signal acquisition device 412' is similar to the wireless signal acquisition device 412 except for different positioning of a third button 454'. Each of a first button 450', a second button 452', a third button 454', and a lead set status indicator 462' are positioned on a front wall 422' of a housing 416'. The first button 450' is positioned above the second button 452'. The third button 454' is positioned below the second button 452' and is configured to be actuated to control an on and off operation of the wireless signal acquisition device 412'. The lead set status indicator 462' is arranged above the first button 450' in a horizontal orientation.

Another embodiment of a wireless signal acquisition device 512 for use with the dock 14 is shown in Fig 16. The description of the wireless signal acquisition device 12 is incorporated by reference to apply to the wireless signal acquisition device 512, except in instances when it conflicts with the specific description and the drawings of the wireless signal acquisition device 512. The wireless signal acquisition device 512 is similar to the wireless signal acquisition device 12 except for a different lead set 530 and a different lead set connector 536. A housing 516 of the wireless signal acquisition device 512 is formed to include a front wall 522, a back wall 524 opposite the front wall 522, and a sidewall 526 extending between and interconnecting the front wall 522 and the back wall 524.

The wireless signal acquisition device 512 includes the lead set 530 removably coupled to the housing 516 as shown in Fig. 16. The lead set 530 includes a first end 532 that interfaces with the wireless signal acquisition device 512 and a second end opposite the first end 532. The first end 532 of the lead set 530 mates with the lead set connector 536 of the wireless signal acquisition device 512. As compared to the wireless signal acquisition device 12, the lead set 530 of the wireless signal acquisition device 512 does not form a portion of the housing 516.

Another embodiment of a wireless signal acquisition device 612 for use with the dock 14 is shown in Figs. 17 and 18. The description of the wireless signal acquisition device 12 is incorporated by reference to apply to the wireless signal acquisition device 612, except in instances when it conflicts with the specific description and the drawings of the wireless signal acquisition device 612. The wireless signal acquisition device 612 includes a housing 616 and a device charging unit 618 as shown in Fig. 17. The housing 616 is formed to include a front wall 622, a back wall 624 opposite the front wall 622, and a sidewall 626 extending between and interconnecting the front wall 622 and the back wall 624 as shown in Fig. 17. Similar to the wireless signal acquisition devices 412, 412', the front wall 622 is positioned inwardly relative to the sidewall 626. In other words, the front wall 622 is recessed relative to the sidewall 626.

The wireless signal acquisition device 612 includes a lead set 630 removably coupled to the housing 616 as shown in Figs. 17 and 18. The lead set 630 includes a first end 632 that mates with a lead set connector 636 that extends inwardly into the housing 616 of the wireless signal acquisition device 612 as shown in Fig. 17. The first end 632 of the lead set 630 extends into the lead set connector 636 to couple the lead set 630 to the housing 616.

The device charging unit 618 of the wireless signal acquisition device 612 includes a rechargeable battery and a power receiver, similar to that of the wireless signal acquisition device 12. The device charging unit 618 is arranged within the housing 616 at an end of the wireless signal acquisition device 612 opposite the lead set connector 636. A ferromagnetic metal component 698B is positioned on or inside of the wireless signal acquisition device 612 near the device charging unit 618.

In some embodiments, the wireless signal acquisition device 612 further includes a first button 650 and a second button 652 as shown in Fig. 17. Each of the first button 650 and the second button 652 are positioned on the front wall 622 of the housing 616. In some embodiments, the wireless signal acquisition device 612 further includes a first biosignal status indicator 656, a second biosignal status indicator 658, a charge status indicator 660, and a lead set status indicator 662. Each of the first biosignal status indicator 656, the second biosignal status indicator 658, the charge status indicator 660, and the lead set status indicator 662 are positioned on the front wall 622 of the housing 616.

The first button 650 is configured to be actuated to begin an ECG exam. In some embodiments, the first biosignal status indicator 656 is arranged on the first button 650 and is configured to illuminate to indicate that the ECG exam is ongoing. The second button 652 is configured to be actuated to begin a rhythm exam. In some embodiments, the second biosignal status indicator 658 is arranged on the second button 652 and is configured to illuminate to indicate that the rhythm exam is ongoing. The second button 652 is arranged below the first button 650.

The charge status indicator 660 is configured to illuminate to indicate a level of charge of the rechargeable battery and/or whether recharging of the rechargeable battery is actively ongoing. For example, the charge status indicator 660 may illuminate while the rechargeable battery is fully charged and/or while the rechargeable battery has a charge level above a predetermined level of charge. As another example, the charge status indicator 660 may flash while the rechargeable battery has a charge level below a predetermined level of charge, which indicates that the rechargeable battery has a low level of charge. The charge status indicator 660 is arranged above the first button 650. The lead set status indicator 662 is configured to illuminate to indicate connection of the first end 632 of the lead set 630 with the lead set connector 636 of the wireless signal acquisition device 612. The lead set status indicator 662 is arranged below the second button 652 in a horizontal orientation.

Another embodiment of a dock 714 is shown in Fig. 19. The description of the dock 14 is incorporated by reference to apply to the dock 714, except in instances when it conflicts with the specific description and the drawings of the dock 714. The dock 714 includes a body 766, a port 768, and a first acquisition-device receiver 770. The first acquisition-device receiver 770 is formed in the body 766 to receive the wireless signal acquisition device 412 therein. Although shown with the wireless signal acquisition device 412, alternative embodiments of the wireless signal acquisition device 12, 12', 12", 212, 212', 312, 312', 412', 512, 612 may be used with the dock 714. The port 768 extends into the body 766 and is configured to receive a power cable therein to provide power to the dock 714.

The body 766 of the dock 714 is formed to include a front wall 772, a back wall 774 opposite the front wall 772, a sleeve wall 775, and a sidewall 776 as shown in Fig. 19. The sleeve wall 775 is in spaced apart relation to the front wall 772. The sidewall 776 extends outwardly away from the front wall 772 to connect to the sleeve wall 775. The sidewall 776 and the sleeve wall 775 cooperate to provide the first acquisition-device receiver 770. The wireless signal acquisition device 412 may be inserted into the first acquisition-device receiver 770 such that a portion of the wireless signal acquisition device 412 is located between the sleeve wall 775 and the front wall 772.

The first acquisition-device receiver 770 comprises a sleeve 780 that allows for insertion of the wireless signal acquisition device 412 from a top open end 780A of the sleeve 780 as suggested in Fig. 19. A bottom closed end 780B of the sleeve 780 opposite the top open end 780A supports the wireless signal acquisition device 412 in the sleeve 780. While the wireless signal acquisition device 412 is positioned in the first acquisition-device receiver 770, the back wall 424 of the wireless signal acquisition device 412 engages a forward-facing surface of the front wall 772.

Similar to the dock 14, the dock 714 includes a dock communication module, a dock pairing module, and a dock charging unit. The charging unit is configured to recharge the rechargeable battery 40 of the device charging unit 18, 618 of the wireless signal acquisition device 412 and is positioned in the sleeve 780 near the bottom closed end 780B so that the dock charging unit of the dock 714 aligns with the device charging unit of the wireless signal acquisition device 412 while the wireless signal acquisition device 412 is positioned in the sleeve 780.

In some embodiments, as shown in Fig. 19, the dock 714 is mountable to a pole 715. The dock 714 is in a vertical orientation while the dock 714 is mounted to the pole 715. The wireless signal acquisition device 412 is in a vertical orientation while the wireless signal acquisition device 412 is inserted into the sleeve 780.

In some embodiments, the dock 714 comprises a lead set holder 794 that is coupled with the pole 715 as shown in Fig. 19. The lead set holder 794 is illustratively formed as a clip and is configured to extend around at least a portion of the lead set while the wireless signal acquisition device 412 is received in the dock 714 to maintain a position of the lead set relative to the dock 714.

A magnet 798A of a positioning assembly is located near the bottom closed end 780B of the sleeve 780 as shown in Fig. 19. The magnet 798A is positioned near the bottom closed end 780B of the sleeve 780 so that the magnet 798A aligns with the ferromagnetic metal component positioned on or in the wireless signal acquisition device 412 while the wireless signal acquisition device 412 is arranged in the sleeve 780. In some embodiments, the magnet 798A may be omitted.

Another embodiment of a dock 814 is shown in Fig. 20. The description of the dock 14 is incorporated by reference to apply to the dock 814, except in instances when it conflicts with the specific description and the drawings of the dock 814. The dock 814 includes a body 866, a port 868, and a first acquisition-device receiver 870. The first acquisition-device receiver 870 is formed in the body 866 to receive the wireless signal acquisition device 412 therein. Although shown with the wireless signal acquisition device 412, alternative embodiments of the wireless signal acquisition device 12, 12', 12", 212, 212', 312, 312', 412', 512, 612 may be used with the dock 814. The port 868 extends into the body 866 and is configured to receive a power cable therein to provide power to the dock 814.

The body 866 of the dock 814 is formed to include a front wall 872, a back wall 874 opposite the front wall 872, a front shelf wall 875, and a top shelf wall 876 as shown in Fig. 20. The front shelf wall 875 is in spaced apart relation to the front wall 872. The top shelf wall 876 extends outwardly away from the front wall 872 to connect to the front shelf wall 875. The first acquisition-device receiver 870 extends downwardly into the body 866 from the top shelf wall 876. The wireless signal acquisition device 412 is inserted into the first acquisition-device receiver 870 as shown in Fig. 20.

The first acquisition-device receiver 870 comprises a pocket 880 having a top open end 880A and a bottom end 880B opposite the top open end 880A as shown in Fig. 20. The bottom end 880B is partially closed such that the bottom end 880B supports the wireless signal acquisition device 412 in the pocket 880 so that the wireless signal acquisition device 412 does not fall through the bottom end 880B.

Similar to the dock 14, the dock 814 includes a dock communication module, a dock pairing module, and a dock charging unit 886. The dock charging unit 886 is positioned in the body 866 near the top open end 880A of the pocket 880 so that the dock charging unit 886 of the dock 814 aligns with the device charging unit of the wireless signal acquisition device 412 while the wireless signal acquisition device 412 is inserted into the pocket 880.

In some embodiments, as shown in Fig. 20, the dock 814 is mountable to a wall in a stationary position. For example, the back wall 874 of the body 866 of the dock 814 is mountable to the wall so that the dock 814 remains stationary relative to the wall. The dock 814 is in a vertical orientation while the dock 814 is mounted to the wall. The wireless signal acquisition device 412 is in a vertical orientation while the wireless signal acquisition device 412 is inserted into the pocket 880.

In some embodiments, the dock 814 includes a lead set holder 894 coupled with the front wall 872 and a lead set groove 892 extending downwardly from the pocket 880 and out of the body 866 of the dock 814 as shown in Fig. 20. The lead set holder 894 is illustratively formed as a clip and is configured to extend around at least a portion of the lead set while the wireless signal acquisition device 412 is received in the dock 814 to maintain a position of the lead set relative to the dock 814. While the wireless signal acquisition device 412 is positioned in the dock 814, a portion of the lead set passes through the lead set groove 892.

A magnet 898A of a positioning assembly is located near the top open end 880A of the pocket 880 as shown in Fig. 20. The magnet 898A is positioned near the top open end 880A of the pocket 880 so that the magnet 898A aligns with the ferromagnetic metal component positioned on or in the wireless signal acquisition device 412 while the wireless signal acquisition device 412 is arranged in the pocket 880. In some embodiments, the magnet 898A may be omitted from the dock 814.

Another embodiment of a dock 814' is shown in Fig. 21. The descriptions of the dock 14 and the dock 814 are incorporated by reference to apply to the dock 814', except in instances when it conflicts with the specific description and the drawings of the dock 814'. In some embodiments, as shown in Fig. 21, the dock 814' is mountable to a wall in a stationary position. The dock 814' includes a body 866', a port 868', a first acquisition-device receiver 870A', a second acquisition-device receiver 870B', and a third acquisition-device receiver 870C'. The acquisition-device receivers 870A', 870B', 870C' are formed in the body 866' to receive the wireless signal acquisition devices 412 therein. Although shown with the wireless signal acquisition device 412, alternative embodiments of the wireless signal acquisition device 12, 12', 12", 212, 212', 312, 312', 412', 512, 612 may be used with the dock 814'. The second acquisition-device receiver 870B' is spaced apart from the first acquisition-device receiver 870A', and the third acquisition-device receiver 870C' is spaced apart from the second acquisition-device receiver 870B'.

The body 866' of the dock 814' is formed to include a front wall 872', a front shelf wall 875', and a top shelf wall 876' as shown in Fig. 21. The front shelf wall 875' is in spaced apart relation to the front wall 872'. The top shelf wall 876' extends outwardly away from the front wall 872' to connect to the front shelf wall 875'. The acquisition-device receivers 870A', 870B', 870C' extend downwardly into the body 866' from the top shelf wall 876'. The wireless signal acquisition devices 412 are inserted into the acquisition-device receivers 870A', 870B', 870C' as shown in Fig. 21.

The wireless signal acquisition device 12, 12', 12", 212, 212', 312, 312', 412, 412', 512, 612 includes a first printed circuit board assembly 102 and a second printed circuit board assembly 104 positioned in the housing 16, 16', 16", 216, 216', 316, 316', 416, 416', 516, 616 as shown in Fig. 22. Though Figs. 22-31B are shown and described as relating to the wireless signal acquisition device 12 and the dock 14, Figs. 22-31B and the corresponding description apply with equal weight to the wireless signal acquisition devices 12', 12", 212, 212', 312, 312', 412, 412', 512, 612 and the docks 714, 814.

Each of the board assemblies 102, 104 is coupled with the housing 16. The first printed circuit board assembly 102 is coupled to the front wall 22, for example, via screws, and the second printed circuit board assembly 104 is coupled to the back wall 24, for example, via screws. The first printed circuit board assembly 102 is arranged between the front wall 22 of the housing 16 and the second printed circuit board assembly 104, and the second printed circuit board assembly 104 is arranged between the first printed circuit board assembly 102 and the back wall 24 of the housing 16.

The first printed circuit board assembly 102 interfaces with the buttons 50, 52 and the biosignal status indicators 56, 58 as shown in Fig. 22. The rechargeable battery 40 is arranged below the first printed circuit board assembly 102 and in front of the second printed circuit board assembly 104 as shown in Fig. 22. The rechargeable battery 40 is coupled to the first printed circuit board assembly 102 via a battery connector 108 as shown in Fig. 24. In some embodiments, the rechargeable battery 40 is positioned in a foam-lined chamber 114 as shown in Fig. 22. A board-to-board interconnect 106 extends between and interconnects the first printed circuit board assembly 102 and the second printed circuit board assembly 104.

The second printed circuit board assembly 104 includes acquisition circuit 120 as shown in Fig. 25. The acquisition circuit 120 acquires the biosignals from the patient via the lead set 30 and digitizes the biosignals. The lead set connector 36 provides a standard interface to lead sets 30 of different channel counts and identification feature(s). The acquisition circuit 120 detects the channel count of the lead set 30 and changes the behavior of the device 12 accordingly. In some embodiments, the acquisition circuit 120 is shielded by a shield can.

As shown in Fig. 22, the wireless signal acquisition device 12 has a thickness T defined between the front wall 22 of the housing 16 and the back wall 24 of the housing 16. In some embodiments, the thickness T of the device 12 is about 20 mm to about 30 mm. In some embodiments, the thickness T of the device 12 is about 25 mm. In some embodiments, the thickness T of the device 12 is not greater than about 25 mm. The wireless signal acquisition device 12 has a width W1 as shown in Fig. 25. In some embodiments, the width W1 of the device 12 is about 40 mm to about 50 mm. In some embodiments, the width W1 of the device 12 is about 42 mm. In some embodiments, the width W1 of the device 12 is not greater than about 50 mm. The thickness T and the width W1 of the device 12 allow the device 12 to be handheld.

As shown in Figs. 25 and 31B, the wireless signal acquisition device 12 includes the device communication module 20, the device pairing module 48, and the device charging unit 18. The dock 14 includes the dock communication module 82, the dock pairing module 84, and the dock charging unit 86 as shown in Figs. 26 and 31A.

The device communication module 20 and the dock communication module 82 allow for data interface between the device 12 and the host device 78 having a real-time (with some margin) wireless link to transmit time accurate data and high-sample-rate buffers. In some embodiments, the device communication module 20 and/or the dock communication module 82 comprise a Bluetooth Low Energy ("BLE") 5.2/5.3+ module. Bluetooth Low Energy allows for isochronous channel communication and encryption mechanisms.

In some embodiments, the device communication module 20 comprises a dual-core Bluetooth Low Energy module that acquires and processes biosignal data while maintaining wireless connection with the dock 14. A dual-core design enables one of the cores (i.e., the wireless data transmitter 44 of the device communication module 20) to wirelessly communicate with the dock 14, while the other core (i.e., the wireless data receiver 46 of the device communication module 20) runs firmware that acquires oversampled biosignal data from the acquisition circuit 120, decimates the data, filters the data, feeds the data through algorithms, and then transmits the data to the wireless communication core (i.e., the wireless data transmitter 44). The wireless communication core (i.e., the wireless data transmitter 44) utilizes a real-time audio functionality of BLE to transmit the data to the dock communication module 82 at deterministic intervals. As shown in Fig. 25, the device communication module 20 is located on the second printed circuit board assembly 104.

In some embodiments, the dock communication module 82 comprises a Bluetooth Low Energy module that receives biosignal data and power feedback data from the wireless data transmitter 44 of the device communication module 20 as suggested in Figs. 31A and 31B. The dock communication module 82 is located on a third printed circuit board assembly 140 of the dock 14 as shown in Fig. 26. In some embodiments, the device communication module 20 and/or the dock communication module 82 comprise Wi-Fi support configured to communicate biosignal data over the hospital network, for example, to a Wi-Fi-enabled display system, such as a PC, a phone, or a tablet.

The device pairing module 48 and the dock pairing module 84 are configured to implement a protocol implementation to prevent cross-compatibility of the device 12 with consumer market devices. In some embodiments, the device pairing module 48 includes the device NFC chip 85, and the dock pairing module 84 includes the dock NFC chip 87. In such an embodiment, the device NFC chip 85 acts as an NFC tag and the dock NFC chip 87 acts as a NFC reader. The NFC link between the pairing modules 48, 84 communicates loop feedback to the dock 14. The NFC chips 85, 87 allow for secure out-of-band secrets exchange and pairing/bonding of the device 12 and the dock 14. Even though the biosignal data communicated between the device 12 and the dock 14 may be anonymous, the pairing modules 48, 84 allow for secure pairing of the device 12 and the dock 14. A wireless link between the communication modules 20, 82 for communication therebetween is formed via out of band pairing with the pairing modules 48, 84. Out of band pairing results in secure pairing between the device 12 and the dock 14. The pairing modules 48, 84 may be used to securely transfer credentials used for the secure pairing. The pairing modules 48, 84 may provide benefits over Bluetooth pairing, such as, for example, speed, simplicity, and low cost. For example, the pairing modules 48, 84 do not require user input in order to pair with one another, and the pairing modules 48, 84 incorporate additional security benefits.

In some embodiments, the device pairing module 48 comprises a NFC tag coil 136 as shown in Figs. 25 and 31B. The NFC tag coil 136 is illustratively formed as a printed coil on the second printed circuit board assembly 104 above the power receiver 42. In some embodiments, the dock pairing module 84 comprises a NFC reader coil 138 as shown in Figs. 26 and 31B. The NFC reader coil 138 is illustratively formed as a printed coil on the third printed circuit board assembly 140 of the dock 14 above the dock charging unit 86. The NFC reader 87 passes an electric current through the NFC reader coil 138, which generates a magnetic field. When the NFC tag 85 and the NFC tag coil 136 are within close proximity to the to the NFC reader 87 and the NFC reader coil 138, the magnetic field induces an electric current within the NFC tag coil 136. The NFC tag coil 136 and the NFC reader coil 138 allow for pairing, and thus, wireless communication between the device 12 and the dock 14.

Illustratively, the pairing modules 48, 84 are only active while the device 12 is positioned in the dock 14 in order to pair the device 12 and the dock 14 and to form the wireless link therebetween. In response to the device 12 being positioned in the dock 14, the device 12 and the dock 14 automatically pair with one another. Once the device 12 and the dock 14 are paired, the wireless link between the device 12 and the dock 14 is automatically established and wireless power transfer from the dock 14 to the device 12 automatically begins.

The dock 14 senses when the device 12 is positioned on the dock 14, so that the dock 14 and the device 12 can be paired. The third printed circuit board assembly 140 of the dock 14 includes two unterminated conductors 142, 144, such as, for example, two capacitive plate electrodes, and a low-power capacitance monitoring circuit 146 as shown in Fig. 26. The low-power capacitance monitoring circuit 146 is configured to detect changes in capacitance between the capacitive plate electrodes 142, 144 while the dock 14 is inactive. The dock 14 remains inactive to conserve power until the device 12 is positioned on the dock 14. The low-power capacitance monitoring circuit 146 is configured to apply a switching waveform to one of the capacitive plate electrodes 142, 144. The low-power capacitance monitoring circuit 146 senses an amount of charge that appears on the other capacitive plate electrode 142, 144 as a result of the excitation to the one of the capacitive plate electrodes 142, 144. The low-power capacitance monitoring circuit 146 wakes up the dock 14 (i.e., the dock communication module 82, the dock charging unit 86, and the dock pairing module 84) when the low-power capacitance monitoring circuit 146 detects a change in capacitance between the capacitive plate electrodes 142, 144. In response to the device 12 being positioned on the dock 14, the device 12 appears to the dock 14 (specifically, the low-power capacitance monitoring circuit 146 of the dock 14) as a large conductive object, which changes the capacitance between the capacitive plate electrodes 142, 144. The low-power capacitance monitoring circuit 146 senses the change in capacitance and wakes up the dock 14. Once woken up, the dock 14 automatically provides an initial minimum power level to the device 12 and initiates the pairing process.

As previously mentioned, the rechargeable battery 40 of the device charging unit 18 of the device 12 is wirelessly recharged by the dock charging unit 86 through inductive charging. The second printed circuit board assembly 104 of the device 12 includes the power receiver 42 of the device charging unit 18 thereon as shown in Fig. 25. Illustratively, the power receiver 42 includes a receiver coil 110 as shown in Figs. 27 and 29. In some embodiments, the device 12 includes a ferrite plate 122 arranged between the rechargeable battery 40 and the receiver coil 110 as shown in Fig. 22. The ferrite plate 122 is configured to shield the device 12 from the inductive magnetic fields and to concentrate the magnetic fields for improved transmitter coil 154 to receiver coil 110 coupling.

The receiver coil 110 is formed to include a plurality of spiral layers 116 as shown in Fig. 29. In the illustrative embodiment, the receiver coil 110 includes six spiral layers 116. In some embodiments, the receiver coil 110 includes between four and ten spiral layers 116. Each of the spiral layers 116 is stacked relative to one another, and each of the spiral layers 116 is equidistant from a central axis C1 of the receiver coil 110 as shown in Fig. 29. In other words, an outer diameter D1 of each of the plurality of spiral layers 116 is the same for each of the plurality of spiral layers 116. As an example, in some embodiments, the outer diameter D1 of each of the plurality of spiral layers 116 is about 40 mm. In some embodiments, the outer diameter D1 of each of the plurality of spiral layers 116 is about 35 mm to about 45 mm. In some embodiments, the outer diameter D1 of each of the plurality of spiral layers 116 is about 25 mm to about 55 mm. Each of the plurality of spiral layers 116 extends circumferentially around the central axis C1. Each of the plurality of spiral layers 116 is separate from one another.

Each of the plurality of spiral layers 116 is made of a flat copper sheet having a rectangular cross-sectional shape (as opposed to a coil having a circular cross-sectional shape). A width W2 of each of the plurality of spiral layers 116 is relatively wide. For example, in some embodiments, the width W2 of each of the plurality of spiral layers 116 is about 5 mm. In some embodiments, the width W2 of each of the plurality of spiral layers 116 is about 4 mm to about 6 mm. In some embodiments, the width W2 of each of the plurality of spiral layers 116 is about 3 mm to about 7 mm. The receiver coil 110 is illustratively a printed circuit board receiver coil 110.

The receiver coil 110 includes a plurality of vias 152 (i.e., vertical interconnect access) as shown in Fig. 29. The plurality of vias 152 extend between and interconnect the plurality of spiral layers 116. The plurality of vias 152 are configured to transfer signals between each layer of the plurality of spiral layers 116 of the receiver coil 110 and allow for electrical connection between each layer of the plurality of spiral layers 116 within the printed circuit board receiver coil 110.

The second printed circuit board assembly 104 comprises a plurality of capacitors 148, a rectifier 118, and a power management and monitoring subsystem 150 as shown in Fig. 31B. The capacitors 148 pass alternating current from the receiver coil 110 to the rectifier 118. The capacitors 148 and the receiver coil 110 are arranged in a parallel configuration. The parallel LC circuit configuration allows for a lower voltage across the receiver coil 110 and for a higher current through the receiver coil 110.

The rectifier 118 is configured to convert the alternating current to direct current. In some embodiments, the rectifier 118 is a Schottky bridge rectifier. Schottky bridge rectifiers have a low voltage drop as compared to other rectifiers, which reduces power loss and heat generation. The power management and monitoring subsystem 150 includes input power protection configured to short out the receiver coil 110 in case of overvoltage conditions. In some embodiments, the power management and monitoring subsystem 150 includes a high-efficiency high-input-voltage step-down converter configured to provide a low-voltage system rail for charging of the rechargeable battery 40. In some embodiments, the power management and monitoring subsystem 150 includes a power path handling circuit configured to select rechargeable battery 40 power or input receiver coil 110 power as an input to downstream circuits. In some embodiments, the power management and monitoring subsystem 150 includes a buck-boost converter configured to up- or down-convert the rechargeable battery 40 voltage or input voltage to a clean digital power rail to power downstream circuits. In some embodiments, the power management and monitoring subsystem 150 includes input receiver coil 110 voltage sensing, rechargeable battery 40 voltage sensing, and device 12 current consumption sensing circuits.

Exemplary circuitry of the device 12 is shown in Figs. 27 and 28. Figs. 27 and 28 are exemplary in nature and represent only one embodiment of circuitry which may be used to control the device 12. It should be understood that any of a number of other circuits may be used to perform the control of the device 12 as described.

In some embodiments, the rechargeable battery 40 is, at a minimum, a 3.7 Wh battery. For example, the rechargeable battery 40 may comprise a 1000 mAh 1S1P lithium-ion polymer battery. Power feedback data may be communicated and/or exchanged with the dock charging unit 86. For example, the device communication module 20 may communicate with the dock communication module 82 and/or the dock charging unit 86 that the receiver coil 110 of the power receiver 42 is receiving too much or too little power. In some embodiments, the rechargeable battery 40 allows for about 14 hours of continuous acquisition of biosignal data at first use. In some embodiments, the rechargeable battery 40 allows for about 8 hours of continuous acquisition of biosignal data at full charge. In some embodiments, the rechargeable battery 40 allows for about 1 hour of continuous acquisition of biosignal data after a 15 minute charge. In some embodiments, the rechargeable battery 40 allows for about 4 hours of continuous acquisition of biosignal data after a one hour charge. In some embodiments, the rechargeable battery 40 allows for about 8 hours of continuous acquisition of biosignal data at an end of life of the rechargeable battery 40. In some embodiments, the rechargeable battery 40 is charged for about 3.5 hours to reach full charge from a zero charge.

The third printed circuit board assembly 140 of the dock 14 includes the dock charging unit 86 as shown in Figs. 26 and 28. Illustratively, the dock charging unit 86 includes a transmitter coil 154 as shown in Fig. 30. The transmitter coil 154 of the dock charging unit 86 receives power from the host device 78 via the port 68. The transmitter coil 154 is powered by a USB 2.0 bus, for example. The transmitter coil 154 generates an electromagnetic field as electric current flows through the transmitter coil 154. While the wireless signal acquisition device 12 is positioned in the recess 80 of the dock 14, an electric current is induced in the receiver coil 110 of the power receiver 42 of the device charging unit 18 of the wireless signal acquisition device 12 due to electromagnetic induction. The electric current received by the receiver coil 110 charges the rechargeable battery 40. Concentric alignment of the receiver coil 110 of the wireless signal acquisition device 12 with the transmitter coil 154 of the dock charging unit 86 allows for and/or maximizes the charging of the rechargeable battery 40. Thus, the transmitter coil 154 of the dock charging unit 86 is positioned on the dock 14 so that the transmitter coil 154 concentrically aligns with the receiver coil 110 of the power receiver 42 of the wireless signal acquisition device 12 while the wireless signal acquisition device 12 is positioned in the recess 80 of the dock 14. Further, the positioning assembly 98 magnetically aligns the wireless signal acquisition device 12 within the first acquisition-device receiver 70 of the dock 14 so that the receiver coil 110 of the power receiver 42 and the transmitter coil 154 of the dock charging unit 86 are concentrically aligned for wireless recharging of the rechargeable battery 40. Due to the wireless charging of the rechargeable battery 40, no galvanic electrical connections are made to the external surrounding, so a 2xMOPP patient isolation barrier is achieved by virtue of the housing 16 isolating Type CF applied parts from any other system.

The transmitter coil 154 is configured the same as the receiver coil 110, as shown in Figs. 29 and 30. The transmitter coil 154 is formed to include a plurality of spiral layers 156. In the illustrative embodiment, the transmitter coil 154 includes six spiral layers 156. In some embodiments, the transmitter coil 154 includes between four and ten spiral layers 156. Each of the spiral layers 156 is stacked relative to one another, and each of the spiral layers 156 is equidistant from a central axis C2 of the transmitter coil 154 as shown in Fig. 30. In other words, an outer diameter D2 of each of the plurality of spiral layers 156 is the same for each of the plurality of spiral layers 156. As an example, the outer diameter D2 of each of the plurality of spiral layers 156 is about 40 mm. In some embodiments, the outer diameter D2 of each of the plurality of spiral layers 156 is about 35 mm to about 45 mm. In some embodiments, the outer diameter D2 of each of the plurality of spiral layers 156 is about 25 mm to about 55 mm. Each of the plurality of spiral layers 156 extends circumferentially around the central axis C2. Each of the plurality of spiral layers 156 is separate from one another.

Each of the plurality of spiral layers 156 is formed of a flat copper sheet having a rectangular cross-sectional shape (as opposed to a coil having a circular cross-sectional shape). A width W3 of each of the plurality of spiral layers 156 is relatively wide. For example, in some embodiments, the width W3 of each of the plurality of spiral layers 156 is about 5 mm. In some embodiments, the width W3 of each of the plurality of spiral layers 156 is about 4 mm to about 6 mm. In some embodiments, the width W3 of each of the plurality of spiral layers 156 is about 3 mm to about 7 mm. The transmitter coil 154 is illustratively a printed circuit board transmitter coil 154.

The transmitter coil 154 includes a plurality of vias 158 (i.e., vertical interconnect access) as shown in Fig. 30. The plurality of vias 158 extend between and interconnect the plurality of spiral layers 156. The plurality of vias 158 are configured to transfer signals between each layer of the plurality of spiral layers 156 of the transmitter coil 154 and allow for electrical connection between each layer of the plurality of spiral layers 156 within the printed circuit board transmitter coil 154.

As shown in Fig. 31A, the transmitter coil 154 of the dock charging unit 86 is connected to a power source (i.e., the host device 78 via the port 68). When an electric current flows through the transmitter coil 154, the transmitter coil 154 generates an electromagnetic field around the transmitter coil 154. When the receiver coil 110 of the power receiver 42 is placed near the electromagnetic field of the transmitter coil 154 of the dock charging unit 86, an electric current is induced in the receiver coil 110 of the power receiver 42 due to electromagnetic induction. The induced current in the receiver coil 110 of the power receiver 42 is an alternating current. The alternating current is converted to direct current using the rectifier 118, as shown in Fig. 31B. The converted direct current is then used to charge the rechargeable battery 40.

Illustratively, the transmitter coil 154 is configured to transfer a maximum of about 1.5 W to the receiver coil 110 of the power receiver 42 while maintaining a low internal ambient temperature of the dock 14 and the device 12. The internal ambient temperature of the device 12 and/or the dock 14 does not rise more than about 10°C during the wireless power transfer. In some embodiments, the internal ambient temperature of the device 12 and/or the dock 14 does not rise more than about 6°C during the wireless power transfer. The relatively low amount of heat generated while transferring as much as 1.5 W to the receiver coil 110 allows for placement of sensitive components in the vicinity of the receiver coil 110 without risk to those sensitive components.

Each of the plurality of spiral layers 116, 156 of the coils 110, 154 is relatively wide to reduce DC and AC resistance effects. The charging units 18, 86 utilize an air-core transformer topology as a means to transfer energy between the transmitter coil 154 of the dock charging unit 86 and the receiver coil 110 of the power receiver 42 of the device charging unit 18. The magnetic field generated by the transmitter coil 154 penetrates the receiver coil 110 through an air gap (the gap between the dock 14 and the device 12). For example, a distance between the coils 110, 154 may be about 6 mm to about 7 mm. A varying magnetic field induces current in the receiver coil 110 of the power receiver 42, which generates a voltage across a receiving load to power the device 12. In some embodiments, each of the coils 110, 154 are made of 0.5 oz copper.

The shape and design of the coils 110, 154 account for the skin effect and the proximity effect commonly seen with traditional coils. The skin effect is the tendency of a current to become distributed within a conductor such that the current density is greatest near the surface (i.e., the skin) of the conductor. Higher-frequency currents have a more pronounced skin effect. Because the skin effect is more pronounced at higher frequencies, the skin effect is not a major concern at the frequencies of the coils 110, 154.

The proximity effect occurs when two or more conductors are placed near one another, and the electromagnetic fields of the conductors interact with one another such that the current in each of the conductors is redistributed to concentrate the current density in the part of the conductor most remote from the interfering conductor. In other words, there is a higher current density in the windings of the conductor closest to the core, which increases the AC resistance of the coil. The proximity effect impacts multilayer windings around a central core (i.e., windings in which each winding gets farther from the central core). Winding the coils 110, 154 in a single layer spiral such that each of the plurality of spiral layers 116, 156 is equidistant from the central axis C1, C2 distributes current evenly across each of the plurality of spiral layers 116, 156 and negates at least a portion of the proximity effect impact. The relatively large surface area (i.e., the width W2, W3) of each of the plurality of spiral layers 116, 156 also helps with the distribution of current.

Increasing the inductance of the coils 110, 154 may increase power transfer efficiency between the coils 110, 154. Inductance may be increased by either increasing the size of the effective loop area of each of the plurality of spiral layers 116, 156 or by increasing the number of turns in the coils 110, 154 (i.e., the number of spiral layers 116, 156). However, increasing the number of spiral layers 116, 156 in the coils 110, 154 increases a length of each of the coils 110, 154, which increases DC resistance of the coils 110, 154. The illustrative six spiral layers 116, 156 allows each of the plurality of spiral layers 116, 165 to be as wide as reasonably possible (the width W2, W3).

The third printed circuit board assembly 140 comprises a plurality of capacitors 162, a boost regulator and transmitter subsystem 164, and a power management and monitoring subsystem 166 as shown in Figs. 26 and 31A. The capacitors 162 and the transmitter coil 154 are arranged in a series configuration. In a series configuration, the Q-factor, which is indicative of efficiency, is proportional to frequency. The higher the frequency, the higher the Q-factor and the higher the efficiency of the power transfer. As previously mentioned, at higher frequencies, the skin and proximity effects may become too drastic. In some embodiments, the target driving frequency is about 1 MHz to about 2 MHz. At these frequencies, the skin effect is not a concern given the relatively small thickness of each of the plurality of spiral layers 116, 156.

The boost regulator and transmitter subsystem 164 is configured to step up input power to a configurable higher voltage, which powers the dock charging unit 86. In some embodiments, the boost regulator and transmitter subsystem 164 includes a MOSFET H-bridge driven by synchronous half-bridge gate drivers. The H-bridge drives the series LC tank circuit formed by the capacitors 162 and the transmitter coil 154. In some embodiments, the drive voltage of the boost regulator and transmitter subsystem 164 is between about 5.5 V and about 8 V. The power management and monitoring subsystem 166 provides input power protection and filtering, steps down input voltage to provide power to the boost regulator and transmitter subsystem 164, and senses input current so that the dock 14 limits the current draw from the host device 78 via the port 68. The power management and monitoring subsystem 166 includes, for example, an over power protector and/or an over current protector.

Exemplary circuitry of the dock 14 is shown in Fig. 28. Fig. 28 is exemplary in nature and represents only one embodiment of circuitry which may be used to control the dock 14. It should be understood that any of a number of other circuits may be used to perform the control of the dock 14 described.

Referring to Fig. 31B, in some embodiments, the device 12 includes a temperature sensor 124 arranged within the device 12 and configured to detect and/or measure an internal temperature of the device 12. In some embodiments, the device 12 includes an accelerometer 126 and/or an inertial measurement unit 128 arranged within the device 12 as suggested in Fig. 31B. The accelerometer 126 is configured to measure an acceleration of the device 12. For example, the measured acceleration of the device 12 may indicate that the device 12 has been picked up from a surface (i.e., removed from the dock 14). In some embodiments, the measured acceleration of the device 12 may be used to determine when to wake up the device 12.

In some embodiments, the device 12 includes an audio unit 130 and/or a buzzer 132 as suggested in Fig. 31B. The audio unit 130 and/or the buzzer 132 are configured to generate a sound. For example, the audio unit 130 and/or the buzzer 132 may generate a sound to serve as an audible indicator, such as a warning. For example, if the temperature sensor 124 detects that the internal temperature of the device 12 is too high, then the audio unit 130 and/or the buzzer 132 may generate a sound. As another example, the audio unit 130 and/or the buzzer 132 may generate a sound when the level of charge of the rechargeable battery 40 is low.

In some embodiments, the device 12 includes an electrically erasable programmable read-only memory (EEPROM) 134 as shown in Fig. 31B. The EEPROM 134 is configured to store various parameters. In some embodiments, the device 12 includes a NOR flash memory 137 configured to store various parameters.

Embodiments of the invention can be described with reference to the following numbered clauses, with preferred features laid out in the dependent clauses:
Clause 1. A biosignal monitoring system comprising a wireless signal acquisition device configured to acquire biosignals of a patient and including a housing and a device charging unit positioned inside of the housing, the device charging unit including a rechargeable battery configured to provide power to the wireless signal acquisition device and a power receiver configured to recharge the rechargeable battery, a dock configured to receive a power cable therein and formed to include a first acquisition-device receiver to receive the wireless signal acquisition device therein, the dock including a dock charging unit positioned inside of the dock, and a positioning assembly configured to mechanically and magnetically align the wireless signal acquisition device within the first acquisition-device receiver of the dock to removably position the wireless signal acquisition device in the first acquisition-device receiver so that the power receiver of the wireless signal acquisition device and the dock charging unit of the dock are aligned for wireless recharging of the rechargeable battery.
Clause 2. The biosignal monitoring system of clause 1, wherein the wireless signal acquisition device is configured to engage wirelessly with the dock and the dock is configured to engage wirelessly with the wireless signal acquisition device, and wherein the wireless signal acquisition device includes a wireless data transmitter configured to transmit biosignal data and power feedback data to the dock, and wherein the dock includes a wireless data receiver configured to receive the biosignal data and the power feedback data from the wireless data transmitter of the wireless signal acquisition device.
Clause 3. The biosignal monitoring system of either clause 1 or 2, wherein the positioning assembly comprises a magnet coupled to the dock and a ferromagnetic metal component coupled to the housing of the wireless signal acquisition device, and wherein the magnet and the ferromagnetic metal component are positioned such that the magnet and the ferromagnetic metal component are aligned and attracted to one another while the wireless signal acquisition device is positioned in the dock.
Clause 4. The biosignal monitoring system of clause 3, wherein the magnet is arranged inside of the dock and the ferromagnetic metal component is arranged inside of the housing of the wireless signal acquisition device.
Clause 5. The biosignal monitoring system of any preceding clauses, wherein the detected biosignals are electrocardiogram signals.
Clause 6. The biosignal monitoring system of any preceding clauses, further comprising a lead set including a first end removably coupled to the housing of the wireless signal acquisition device and a second end opposite the first end and including a plurality of wire leads configured to be coupled to electrodes positioned on the patient.
Clause 7. The biosignal monitoring system of clause 6, wherein the dock is formed to include a lead set holder configured to extend around at least a portion of the lead set while the wireless signal acquisition device is received in the dock to maintain a position of the lead set relative to the dock.
Clause 8. The biosignal monitoring system of either clause 6 or 7, wherein the second end provides a 12 lead diagnostic signal.
Clause 9. The biosignal monitoring system of either clause 6 or 7, wherein the second end provides a 15 lead diagnostic signal.
Clause 10. The biosignal monitoring system of either clause 6 or 7, wherein the second end provides a 5 lead diagnostic signal.
Clause 11. The biosignal monitoring system of either clause 6 or 7, wherein the second end provides a 3 lead diagnostic signal.
Clause 12. The biosignal monitoring system of any preceding clauses, wherein the wireless signal acquisition device includes a first button positioned on the housing and configured to be actuated to start an electrocardiogram exam.
Clause 13. The biosignal monitoring system of clause 12, wherein the wireless signal acquisition device includes a biosignal status indicator extending around a perimeter of the first button and configured to illuminate to indicate acquisition of the biosignals during the electrocardiogram exam.
Clause 14. The biosignal monitoring system of clause 13, wherein the wireless signal acquisition device includes a second button positioned on the housing and configured to be actuated to start a rhythm exam.
Clause 15. The biosignal monitoring system of clause 14, wherein the wireless signal acquisition device includes a biosignal status indicator extending around a perimeter of the second button and configured to illuminate to indicate acquisition of the biosignals during the rhythm exam.
Clause 16. The biosignal monitoring system of clause 15, wherein the wireless signal acquisition device includes a third button positioned on the housing and configured to be actuated to power on, to power off, and to wake up the wireless signal acquisition device.
Clause 17. The biosignal monitoring system of any preceding clauses, wherein the wireless signal acquisition device includes a charge status indicator positioned on the housing, and wherein the charge status indicator is configured to illuminate to indicate a level of charge of the rechargeable battery.
Clause 18. The biosignal monitoring system of clause 17, further comprising a lead set removably coupled to the housing, and wherein the wireless signal acquisition device includes a lead set status indicator positioned on the housing and configured to illuminate to indicate connection of the lead set with the housing of the wireless signal acquisition device.
Clause 19. The biosignal monitoring system of any preceding clauses, wherein the housing of the wireless signal acquisition device defines a front wall, a back wall opposite the front wall, and a sidewall extending between and interconnecting the front wall and the back wall.
Clause 20. The biosignal monitoring system of clause 19, wherein the back wall of the wireless signal acquisition device engages a forwardly-facing surface of the first acquisition-device receiver while the wireless signal acquisition device is positioned in the first acquisition-device receiver of the dock.
Clause 21. The biosignal monitoring system of either clause 19 or 20, wherein an entirety of the back wall of the wireless signal acquisition device engages the dock while the wireless signal acquisition device is positioned in the first acquisition-device receiver of the dock and the front wall of the wireless signal acquisition device is visible while the wireless signal acquisition device is positioned in the dock.
Clause 22. The biosignal monitoring system of any one of clauses 19 to 21, wherein the dock includes a front wall and a sidewall coupled to the front wall, and wherein the first acquisition-device receiver comprises a recess that extends inwardly into the dock from the front wall.
Clause 23. The biosignal monitoring system of clause 22, wherein the recess is defined by a forwardly-facing surface and a side surface extending between and interconnecting the forwardly-facing surface of the recess and the front wall of the dock.
Clause 24. The biosignal monitoring system of clause 23, wherein the forwardly-facing surface of the recess is parallel to the front wall of the housing of the wireless signal acquisition device while the wireless signal acquisition device is positioned in the first acquisition-device receiver of the dock.
Clause 25. The biosignal monitoring system of clause 22, wherein the dock is formed to include a lead set groove extending outwardly from the recess to receive a portion of the wireless signal acquisition device therein while the wireless signal acquisition device is positioned in the first acquisition-device receiver of the dock.
Clause 26. The biosignal monitoring system of any one of clauses 22 to 25, wherein the dock is formed to include a grip that extends outwardly from the recess to facilitate gripping of the housing of the wireless signal acquisition device to remove the wireless signal acquisition device from the dock.
Clause 27. The biosignal monitoring system of clause 26, wherein the grip includes a first grip groove and a second grip groove opposite the first grip groove, each of the first grip groove and the second grip groove extends outwardly from the recess.
Clause 28. The biosignal monitoring system of any preceding clauses, wherein the dock is mountable to a wall in a stationary position.
Clause 29. The biosignal monitoring system of clause 28, wherein a forwardly-facing surface of the first acquisition-device receiver is parallel to the wall while the dock is mounted to the wall.
Clause 30. The biosignal monitoring system of any preceding clauses, wherein the dock is configured to be supported on a horizontal surface while the dock is in use.
Clause 31. The biosignal monitoring system of any preceding clauses, wherein the dock is mountable to a pole and the dock is stationary relative to the pole while the dock is mounted to the pole.
Clause 32. The biosignal monitoring system of any preceding clauses, wherein the dock is formed to include a second acquisition-device receiver spaced apart from the first acquisition-device receiver.
Clause 33. The biosignal monitoring system of clause 32, wherein the dock is formed to include a third acquisition-device receiver spaced apart from the second acquisition-device receiver.
Clause 34. A wireless signal acquisition device configured to acquire biosignals of a patient and configured to wirelessly communicate with a dock, the wireless signal acquisition device comprising a housing including a front wall, a back wall opposite the front wall, and a sidewall extending between and interconnecting the front wall and the back wall, the front wall, the back wall, and the sidewall cooperate to form an electronics receiving-space, a device charging unit positioned in the electronics receiving-space of the housing, the device charging unit including a rechargeable battery configured to provide power to the wireless signal acquisition device and a power receiver configured to recharge the rechargeable battery, and a docking positioner coupled to the housing and configured to aid magnetic alignment and biasing of the wireless signal acquisition device within the dock so that the power receiver of the device charging unit of the wireless signal acquisition device is aligned with the dock for wireless recharging of the rechargeable battery.
Clause 35. The wireless signal acquisition device of clause 34, wherein the docking positioner comprises a ferromagnetic metal component.
Clause 36. The wireless signal acquisition device of either clause 34 or 35, wherein the detected biosignals are electrocardiogram signals.
Clause 37. The wireless signal acquisition device of any one of clauses 34 to 36, further comprising a lead set including a first end removably coupled to the housing and a second end opposite the first end.
Clause 38. The wireless signal acquisition device of clause 37, wherein the docking positioner and the lead set are positioned at opposing ends of the wireless signal acquisition device.
Clause 39. The wireless signal acquisition device of either clause 37 or 38, further comprising a lead set status indicator positioned on the housing, and wherein the lead set status indicator is configured to illuminate to indicate connection of the first end of the lead set with the housing.
Clause 40. The wireless signal acquisition device of any one of clauses 37 to 39, wherein the second end provides a 12 lead diagnostic signal.
Clause 41. The wireless signal acquisition device of any one of clauses 37 to 39, wherein the second end provides a 15 lead diagnostic signal.
Clause 42. The wireless signal acquisition device of any one of clauses 37 to 39, wherein the second end provides a 5 lead diagnostic signal.
Clause 43. The wireless signal acquisition device of any one of clauses 37 to 39, wherein the second end provides a 3 lead diagnostic signal.
Clause 44. The wireless signal acquisition device of any one of clauses 34 to 43, further comprising a first button positioned on the housing and configured to be actuated to start an electrocardiogram exam.
Clause 45. The wireless signal acquisition device of clause 44, further comprising a biosignal status indicator extending around a perimeter of the first button and configured to illuminate to indicate acquisition of the biosignals during the electrocardiogram exam.
Clause 46. The wireless signal acquisition device of either clause 44 or 45, further comprising a second button positioned on the housing and configured to be actuated to start a rhythm exam.
Clause 47. The wireless signal acquisition device of clause 46, further comprising a biosignal status indicator extending around a perimeter of the second button and configured to illuminate to indicate acquisition of the biosignals during the rhythm exam.
Clause 48. The wireless signal acquisition device of either clause 42 or 47, further comprising a third button positioned on the housing and configured to be actuated to power on, to power off, and to wake up the wireless signal acquisition device.
Clause 49. The wireless signal acquisition device of any one of clauses 34 to 48, further comprising a charge status indicator positioned on the housing, and wherein the charge status indicator is configured to illuminate to indicate a level of charge of the rechargeable battery.
Clause 50. A dock configured to engage wirelessly with a wireless signal acquisition device, the dock comprising a body formed to include a first acquisition-device receiver to receive the wireless signal acquisition device therein, a port extending into the body and configured to receive a power cable therein to provide power to the dock, a charging unit positioned inside the body of the dock, and a docking positioner coupled to the body and configured to magnetically align the wireless signal acquisition device within the dock so that the charging unit of the dock is aligned with the wireless signal acquisition device for wireless recharging of the wireless signal acquisition device.
Clause 51. The dock of clause 50, wherein the docking positioner comprises a static magnet and a steel shunt that receives the static magnet therein so that a magnetic field of the static magnet is concentrated near an outwardly facing surface of the static magnet.
Clause 52. The dock of either clause 50 or 51, wherein the body of the dock includes a front wall and a sidewall coupled to the front wall, and wherein the first acquisition-device receiver comprises a recess that extends inwardly into the body from the front wall.
Clause 53. The dock of clause 52, wherein the recess is defined by a forwardly-facing surface and a side surface extending between and interconnecting the forwardly-facing surface of the recess and the front wall of the body, and wherein the dock further comprises a power availability status indicator positioned on the forwardly-facing surface of the recess and configured to indicate a power availability of the dock.
Clause 54. The dock of either clause 52 or 53, wherein the body is formed to include a lead set groove extending outwardly from the recess, and wherein the lead set groove and the docking positioner are positioned on opposing ends of the dock.
Clause 55. The dock of clause 54, wherein the body is formed to include a grip that extends outwardly from the recess.
Clause 56. The dock of clause 55, wherein the grip includes a first grip groove and a second grip groove opposite the first grip groove, and wherein each of the first grip groove and the second grip groove extends outwardly from the recess.
Clause 57. The dock of any one of clauses 50 to 56, wherein the dock is mountable to a wall.
Clause 58. The dock of clause 57, wherein a forwardly-facing surface of the first acquisition-device receiver is parallel to the wall while the dock is mounted to the wall.
Clause 59. The dock of any one of clauses 50 to 58, wherein the dock is configured to be supported on a horizontal surface while the dock is in use.
Clause 60. The dock of any one of clauses 50 to 59, wherein the dock is mountable to a pole and the dock is stationary relative to the pole while the dock is mounted to the pole.
Clause 61. The dock of any one of clauses 50 to 60, wherein the body of the dock includes a front wall, a sleeve wall in spaced apart relation to the front wall, and a sidewall extending outwardly from the front wall and interconnecting the front wall and the sleeve wall, and wherein the front wall, the sidewall, and the sleeve wall cooperate to provide the first acquisition-device receiver.
Clause 62. The dock of clause 61, wherein the dock is formed to include a lead set holder configured to extend around at least a portion of the wireless signal acquisition device to maintain a position of the wireless signal acquisition device relative to the dock while the wireless signal acquisition device is positioned in the dock.
Clause 63. The dock of either clause 61 or 62, wherein the body is formed to include a lead set groove extending outwardly from the first acquisition-device receiver.
Clause 64. The dock of any one of clauses 50 to 63, wherein the body is formed to include a second acquisition-device receiver spaced apart from the first acquisition-device receiver.
Clause 65. The dock of clause 64, wherein the body is formed to include a third acquisition-device receiver spaced apart from the second acquisition-device receiver.
Clause 66. The dock of any one of clauses 50 to 65, wherein the dock is integrated with a cardiograph.
Clause 67. A biosignal monitoring system comprising a wireless signal acquisition device configured to acquire biosignals of a patient and including a device charging unit positioned inside of the wireless signal acquisition device, the device charging unit including a rechargeable battery configured to provide power to the wireless signal acquisition device and a power receiver in electrical communication with the rechargeable battery and having a receiver coil configured to recharge the rechargeable battery, the receiver coil defined by a plurality of spiral layers that each extend circumferentially around a central axis of the receiver coil, wherein each of the plurality of spiral layers is equidistant from the central axis of the receiver coil, and a dock configured to receive the wireless signal acquisition device therein and wirelessly engage with the wireless signal acquisition device, the dock including a dock charging unit positioned inside of the dock and including a transmitter coil, the transmitter coil defined by a plurality of spiral layers that each extend circumferentially around a central axis of the transmitter coil, and wherein each of the plurality of spiral layers of the transmitter coil is equidistant from the central axis of the transmitter coil, wherein, in response to the receiver coil being concentrically aligned with the transmitter coil while the wireless signal acquisition device is positioned in the dock, an electric current is induced in the receiver coil due to electromagnetic induction so that the rechargeable battery is wirelessly recharged via inductive resonant charging.
Clause 68. The system of clause 67, wherein the wireless signal acquisition device includes a device communication module having a wireless data transmitter and a wireless data receiver, and wherein the wireless data receiver is configured to acquire the biosignals and communicate the biosignals to the wireless data transmitter, and the wireless data transmitter is configured to wirelessly transmit the biosignals to the dock.
Clause 69. The system of clause 68, wherein the dock includes a dock communication module configured to wirelessly receive the biosignals from the wireless data transmitter of the device communication module.
Clause 70. The system of either clause 68 or 69, wherein the wireless data transmitter of the device communication module is configured to wirelessly transmit power feedback data to the dock communication module indicative of a power level of the rechargeable battery.
Clause 71. The system of any one of clauses 67 to 70, wherein the wireless signal acquisition device includes a device pairing module positioned inside of the wireless signal acquisition device and the dock includes a dock pairing module positioned inside of the dock, and wherein the device pairing module and the dock pairing module communicate with one another to form a wireless link between the device communication module and the dock communication module for wireless communication therebetween.
Clause 72. The system of clause 71, wherein the device pairing module and the dock pairing module automatically communicate with one another to form the wireless link in response to the wireless signal acquisition device being positioned in the dock.
Clause 73. The system of clause 71, wherein the device pairing module acts as an active near-field communication tag and the dock pairing module acts as a near-field communication reader.
Clause 74. The system of clause 71, wherein the device pairing module includes a near-field communication tag coil and the dock pairing module includes a near-field communication reader coil.
Clause 75. The system of clause 74, wherein the near-field communication tag coil is fabricated as part of a first printed circuit board assembly of the wireless signal acquisition device and the near-field communication reader coil is fabricated as part of a second printed circuit board assembly of the dock.
Clause 76. The system of any one of clauses 67 to 75, wherein wireless recharging of the rechargeable battery automatically occurs in response to the wireless signal acquisition device being placed in the dock.
Clause 77. The system of any one of clauses 67 to 76, wherein the dock is coupled to a host device to receive power therefrom and to provide power to the dock charging unit.
Clause 78. The system of any one of clauses 67 to 77, wherein each of the plurality of spiral layers of the receiver coil is made of a flat copper sheet.
Clause 79. The system of any one of clauses 67 to 78, wherein the receiver coil includes a plurality of vias that extend between and interconnect each of the plurality of spiral layers of the receiver coil to transfer signals between each of the plurality of spiral layers of the receiver coil.
Clause 80. The system of any one of clauses 67 to 79, wherein each of the plurality of spiral layers of the transmitter coil is made of a flat copper sheet.
Clause 81. The system of any one of clauses 67 to 80, wherein the transmitter coil includes a plurality of vias that extend between and interconnect each of the plurality of spiral layers of the transmitter coil to transfer signals between each of the plurality of spiral layers of the transmitter coil.
Clause 82. The system of any one of clauses 67 to 81, wherein the wireless signal acquisition device includes a ferrite plate arranged between the rechargeable battery and the receiver coil.
Clause 83. A control system for a biosignal monitoring system comprising a device communication module arranged in a wireless signal acquisition device of the biosignal monitoring system and configured to acquire biosignals of a patient, the device communication module including a wireless data transmitter configured to wirelessly communicate the biosignals to a dock and a wireless data receiver configured to acquire the biosignals and communicate the biosignals to the wireless data transmitter, a dock communication module arranged in the dock of the biosignal monitoring system and configured to wirelessly receive the biosignals from the wireless data transmitter of the device communication module, and a pairing module assembly configured to securely pair the device communication module and the dock communication module to form a wireless link for wireless communication therebetween, the pairing module assembly including a device pairing module arranged in the wireless signal acquisition device and a dock pairing module arranged in the dock, wherein the wireless link is automatically formed in response to the wireless signal acquisition device being positioned in the dock.
Clause 84. The control system of clause 83, further comprising a device charging unit positioned inside of the wireless signal acquisition device to power the wireless signal acquisition device and a dock charging unit positioned inside of the dock to wirelessly recharge the device charging unit while the wireless signal acquisition device is positioned in the dock.
Clause 85. The control system of clause 84, wherein the device charging unit includes a rechargeable battery configured to provide power to the wireless signal acquisition device and a power receiver in electrical communication with the rechargeable battery and having a receiver coil configured to recharge the rechargeable battery.
Clause 86. The control system of clause 85, wherein the wireless data transmitter of the device communication module of the wireless signal acquisition device is configured to wirelessly communicate power feedback data to the dock communication module indicative of a power level of the rechargeable battery.
Clause 87. The control system of either clause 85 or 86, wherein the receiver coil is defined by a plurality of spiral layers that each extend circumferentially around a central axis of the receiver coil, and wherein each of the plurality of spiral layers is equidistant from the central axis of the receiver coil.
Clause 88. The control system of clause 87, wherein each of the plurality of spiral layers of the receiver coil is made of a flat copper sheet.
Clause 89. The control system of any one of clauses 85 to 88, wherein the dock charging unit includes a transmitter coil defined by a plurality of spiral layers that each extend circumferentially around a central axis of the transmitter coil, and wherein each of the plurality of spiral layers of the transmitter coil is equidistant from the central axis of the transmitter coil, and wherein, in response to the receiver coil being aligned with the transmitter coil while the wireless signal acquisition device is positioned in the dock, an electric current is induced in the receiver coil due to electromagnetic induction so that the rechargeable battery is wirelessly recharged via inductive resonant charging.
Clause 90. The control system of clause 89, wherein the plurality of spiral layers of the transmitter coil is made of a flat copper sheet.
Clause 91. The control system of either clause 89 or 90, wherein the dock is coupled to a host device to receive power therefrom and to provide power to the dock charging unit, and wherein the dock communication module communicates the biosignals to the host device.
Clause 92. The control system of any one of clauses 83 to 91, wherein the device pairing module acts as a near-field communication tag and the dock pairing module acts as a near-field communication reader.
Clause 93. The control system of clause 92, wherein the device pairing module includes a near-field communication tag coil and the dock pairing module includes a near-field communication reader coil.
Clause 94. The control system of clause 93, wherein the near-field communication tag coil is fabricated as part of a first printed circuit board assembly of the wireless signal acquisition device and the near-field communication reader coil is fabricated as part of a second printed circuit board assembly of the dock.
Clause 95. A printed circuit board coil for use in inductive resonant charging of a wireless biosignal acquisition device comprising a plurality of spiral layers through which an alternating current flows, the plurality of spiral layers each extend circumferentially around a central axis of the printed circuit board coil and each of the plurality of spiral layers are axially spaced apart from one another, a plurality of vias that extend between and interconnect each of the plurality of spiral layers to transfer signals between each of the plurality of spiral layers, and a rectifier configured to convert the alternating current into a direct current, wherein each spiral layer of the plurality of spiral layers has an outer diameter that is the same for each spiral layer to cause the alternating current flowing through the plurality of spiral layers to be evenly distributed throughout each spiral layer of the plurality of spiral layers.
Clause 96. The printed circuit board coil of clause 95, wherein each spiral layer of the plurality of spiral layers is made of a flat copper sheet.
Clause 97. The printed circuit board coil of either clause 95 or 96, wherein the plurality of spiral layers includes at least four layers.
Clause 98. A biosignal monitoring system comprising a wireless signal acquisition device configured to acquire biosignals of a patient and including a device pairing module positioned inside of the wireless signal acquisition device, and a dock configured to receive the wireless signal acquisition device therein and wirelessly engage with the wireless signal acquisition device, the dock including a dock pairing module positioned inside of the dock and a printed circuit board assembly, the dock pairing module is configured to pair with the device pairing module to establish a wireless link between the wireless signal acquisition device and the dock for wireless communication therebetween, and the printed circuit board assembly includes a first capacitive plate electrode, a second capacitive plate electrode, and a capacitance monitoring circuit configured to detect a capacitance between the first capacitive plate electrode and the second capacitive plate electrode, wherein, in response to the wireless signal acquisition device being positioned on the dock, the capacitance monitoring circuit detects a change in the capacitance between the first capacitive plate electrode and the second capacitive plate electrode, and wherein, in response to the change in the capacitance, the capacitance monitoring circuit initiates pairing between the device pairing module and the dock pairing module to establish the wireless link.
Clause 99. The system of clause 98, wherein the wireless signal acquisition device includes a device charging unit positioned inside of the wireless signal acquisition device, the device charging unit including a rechargeable battery configured to provide power to the wireless signal acquisition device and a power receiver having a receiver coil configured to recharge the rechargeable battery.
Clause 100. The system of clause 99, wherein the receiver coil is defined by a plurality of spiral layers that each extend circumferentially around a central axis of the receiver coil, and wherein each of the plurality of spiral layers is equidistant from the central axis of the receiver coil.
Clause 101. The system of either clause 99 or 100, wherein the dock includes a dock charging unit positioned inside of the dock and including a transmitter coil.
Clause 102. The system of clause 101, wherein the transmitter coil is defined by a plurality of spiral layers that each extend circumferentially around a central axis of the transmitter coil, and wherein each of the plurality of spiral layers of the transmitter coil is equidistant from the central axis of the transmitter coil.
Clause 103. The system of either clause 101 or 102, wherein, in response to the change in the capacitance, the transmitter coil of the dock charging unit automatically provides an initial minimum power amount to the receiver coil of the device charging unit.
Clause 104. The system of any one of clauses 98 to 103, wherein the device pairing module acts as a near-field communication tag and the dock pairing module acts as a near-field communication reader.
Clause 105. The system of clause 104, wherein the device pairing module includes a near-field communication tag coil and the dock pairing module includes a near-field communication reader coil, and wherein the near-field communication reader coil is fabricated as part of a printed circuit board of the printed circuit board assembly of the dock.
Clause 106. A wireless signal acquisition device comprising a housing, an electric circuit carried by the housing, and a plurality of wire leads coupled to the electric circuit and extending from the housing, wherein the electric circuit includes a rechargeable battery that is recharged via inductive resonant recharging, a communication module that wirelessly transmits acquired ECG data according to a wireless communication protocol, and a pairing module that wirelessly pairs the communication module with an external device according to a wireless pairing protocol.
Clause 107. The wireless signal acquisition device of clause 106, wherein the communication module comprises a Bluetooth Low Energy (BLE) transmitter.
Clause 108. The wireless signal acquisition device of clause 106, wherein the wireless communication protocol is a BLE protocol.
Clause 109. The wireless signal acquisition device of clause 106, wherein the pairing module comprises a near field communication (NFC) chip.
Clause 110. The wireless signal acquisition device of clause 106, wherein the wireless pairing protocol is an NFC protocol.
Clause 111. A biosignal monitoring system comprising a signal acquisition device configured to acquire biosignals of a patient and including a housing and a device charging unit positioned inside of the housing, the device charging unit including a rechargeable battery configured to provide power to the signal acquisition device and a power receiver configured to recharge the rechargeable battery, a dock configured to receive a power cable and formed to include a acquisition-device receiver to receive the signal acquisition device therein, the dock including a dock charging unit positioned inside of the dock, and a positioning assembly configured to mechanically and magnetically align the signal acquisition device within the acquisition-device receiver of the dock to removably position the signal acquisition device in the acquisition-device receiver so that the power receiver of the signal acquisition device and the dock charging unit of the dock are aligned for wireless recharging of the rechargeable battery.
Clause 112. The biosignal monitoring system of clause 111, wherein the positioning assembly comprises a magnet coupled to the dock and a ferromagnetic metal component coupled to the housing of the signal acquisition device, and wherein the magnet and the ferromagnetic metal component are positioned such that the magnet and the ferromagnetic metal component are aligned and attracted to one another while the signal acquisition device is positioned in the dock.
Clause 113. The biosignal monitoring system of clause 112, wherein the magnet is arranged inside of the dock and the ferromagnetic metal component is arranged inside of the housing of the signal acquisition device.
Clause 114. The biosignal monitoring system of any one of clauses 111 to 113, further comprising a lead set including a first end removably coupled to the housing of the signal acquisition device and a second end opposite the first end and including a plurality of wire leads configured to be coupled to electrodes positioned on the patient.
Clause 115. The biosignal monitoring system of clause 114, wherein the positioning assembly and the lead set are positioned at opposing ends of the signal acquisition device.
Clause 116. The biosignal monitoring system of either clause 114 or 115, wherein the signal acquisition device includes a charge status indicator on the housing and a lead set status indicator on the housing, and wherein the charge status indicator is configured to illuminate to indicate a level of charge of the rechargeable battery and the lead set status indicator is configured to illuminate to indicate connection of the lead set with the housing of the signal acquisition device.
Clause 117. The biosignal monitoring system of any one of clauses 111 to 116, wherein the housing of the signal acquisition device defines a front wall, a back wall opposite the front wall, and a sidewall extending between and interconnecting the front wall and the back wall, and wherein the back wall of the signal acquisition device engages a forwardly-facing surface of the acquisition-device receiver while the signal acquisition device is positioned in the acquisition-device receiver of the dock.
Clause 118. The biosignal monitoring system of clause 117, wherein the dock includes a front wall and a sidewall coupled to the front wall, and wherein the acquisition-device receiver comprises a recess that extends inwardly into the dock from the front wall, and wherein the recess is defined by the forwardly-facing surface and a side surface extending between and interconnecting the forwardly-facing surface of the recess and the front wall of the dock.
Clause 119. The biosignal monitoring system of any one of clauses 111 to 118, wherein the signal acquisition device is configured to engage wirelessly with the dock and the dock is configured to engage wirelessly with the signal acquisition device, and wherein the signal acquisition device includes a wireless data transmitter configured to transmit biosignal data and power feedback data to the dock, and wherein the dock includes a wireless data receiver configured to receive the biosignal data and the power feedback data from the wireless data transmitter of the signal acquisition device.
Clause 120. The biosignal monitoring system of any one of clauses 111 to 119, wherein the power receiver of the device charging unit includes a receiver coil configured to recharge the rechargeable battery, the receiver coil defined by a plurality of spiral layers that each extend circumferentially around a central axis of the receiver coil, and wherein each of the plurality of spiral layers is equidistant from the central axis of the receiver coil.
Clause 121. The biosignal monitoring system of clause 120, wherein the dock charging unit includes a transmitter coil defined by a plurality of spiral layers that each extend circumferentially around a central axis of the transmitter coil, and wherein each of the plurality of spiral layers of the transmitter coil is equidistant from the central axis of the transmitter coil.
Clause 122. The biosignal monitoring system of clause 121, wherein in response to the receiver coil being concentrically aligned with the transmitter coil while the signal acquisition device is positioned in the dock, an electric current is induced in the receiver coil due to electromagnetic induction so that the rechargeable battery is wirelessly recharged via inductive resonant charging.
Clause 123. The biosignal monitoring system of any one of clauses 111 to 122, wherein the dock is mountable to a support structure in a stationary position, and wherein a forwardly-facing surface of the acquisition-device receiver is parallel to the support structure while the dock is mounted to the support structure.
Clause 124. The biosignal monitoring system of clause 123, wherein the support structure includes a pole or a wall.
Clause 125. The biosignal monitoring system of any one of clauses 111 to 124, wherein the detected biosignals are electrocardiogram signals.
Clause 126. A method of using a biosignal monitoring system, the method comprising: acquiring biosignals of a patient using a signal acquisition device, positioning the signal acquisition device in an acquisition-device receiver of a dock, magnetically biasing the signal acquisition device within the acquisition-device receiver of the dock to align a power receiver of the signal acquisition device with a power transmitter of the dock, and wirelessly recharging a rechargeable battery of the signal acquisition device via inductive resonant charging.
Clause 127. The method of clause 126, further comprising coupling a first lead set to the signal acquisition device before acquiring the biosignals, and further comprising removing the first lead set from the signal acquisition device and coupling a second lead set to the signal acquisition device, the first lead set having a first number of leads and the second lead set having a second number of leads different than the first number of leads.
Clause 128. The method of either clause 126 or 127, further comprising concentrically aligning a receiver coil of the power receiver and a transmitter coil of the power transmitter of the dock and inducing an electric current in the receiver coil of the power receiver to wirelessly recharge the rechargeable battery.
Clause 129. The method of any one of clauses 126 to 128, further comprising automatically wirelessly recharging the rechargeable battery in response to the signal acquisition device being positioned in the acquisition-device receiver of the dock.
Clause 130. The method of any one of clauses 126 to 129, further comprising simultaneously acquiring the biosignals and wirelessly transmitting biosignal data and power feedback data to the dock from the signal acquisition device.

## Claims

1. A biosignal monitoring system comprising
a signal acquisition device configured to acquire biosignals of a patient and including a housing and a device charging unit positioned inside of the housing, the device charging unit including a rechargeable battery configured to provide power to the signal acquisition device and a power receiver configured to recharge the rechargeable battery,
a dock configured to receive a power cable and formed to include a acquisition-device receiver to receive the signal acquisition device therein, the dock including a dock charging unit positioned inside of the dock, and
a positioning assembly configured to mechanically and magnetically align the signal acquisition device within the acquisition-device receiver of the dock to removably position the signal acquisition device in the acquisition-device receiver so that the power receiver of the signal acquisition device and the dock charging unit of the dock are aligned for wireless recharging of the rechargeable battery.

2. The biosignal monitoring system of claim 1, wherein the positioning assembly comprises a magnet coupled to the dock and a ferromagnetic metal component coupled to the housing of the signal acquisition device, and wherein the magnet and the ferromagnetic metal component are positioned such that the magnet and the ferromagnetic metal component are aligned and attracted to one another while the signal acquisition device is positioned in the dock.

3. The biosignal monitoring system of claim 2, wherein the magnet is arranged inside of the dock and the ferromagnetic metal component is arranged inside of the housing of the signal acquisition device.

4. The biosignal monitoring system of any preceding claims, further comprising a lead set including a first end removably coupled to the housing of the signal acquisition device and a second end opposite the first end and including a plurality of wire leads configured to be coupled to electrodes positioned on the patient.

5. The biosignal monitoring system of claim 4, wherein the positioning assembly and the lead set are positioned at opposing ends of the signal acquisition device.

6. The biosignal monitoring system of either claim 4 or 5, wherein the signal acquisition device includes a charge status indicator on the housing and a lead set status indicator on the housing, and wherein the charge status indicator is configured to illuminate to indicate a level of charge of the rechargeable battery and the lead set status indicator is configured to illuminate to indicate connection of the lead set with the housing of the signal acquisition device.

7. The biosignal monitoring system of any preceding claims, wherein the housing of the signal acquisition device defines a front wall, a back wall opposite the front wall, and a sidewall extending between and interconnecting the front wall and the back wall, and wherein the back wall of the signal acquisition device engages a forwardly-facing surface of the acquisition-device receiver while the signal acquisition device is positioned in the acquisition-device receiver of the dock.

8. The biosignal monitoring system of claim 7, wherein the dock includes a front wall and a sidewall coupled to the front wall, and wherein the acquisition-device receiver comprises a recess that extends inwardly into the dock from the front wall, and wherein the recess is defined by the forwardly-facing surface and a side surface extending between and interconnecting the forwardly-facing surface of the recess and the front wall of the dock.

9. The biosignal monitoring system of any preceding claims, wherein the signal acquisition device is configured to engage wirelessly with the dock and the dock is configured to engage wirelessly with the signal acquisition device, and wherein the signal acquisition device includes a wireless data transmitter configured to transmit biosignal data and power feedback data to the dock, and wherein the dock includes a wireless data receiver configured to receive the biosignal data and the power feedback data from the wireless data transmitter of the signal acquisition device.

10. The biosignal monitoring system of any preceding claims, wherein the power receiver of the device charging unit includes a receiver coil configured to recharge the rechargeable battery, the receiver coil defined by a plurality of spiral layers that each extend circumferentially around a central axis of the receiver coil, and wherein each of the plurality of spiral layers is equidistant from the central axis of the receiver coil.

11. The biosignal monitoring system of claim 10, wherein the dock charging unit includes a transmitter coil defined by a plurality of spiral layers that each extend circumferentially around a central axis of the transmitter coil, and wherein each of the plurality of spiral layers of the transmitter coil is equidistant from the central axis of the transmitter coil.

12. The biosignal monitoring system of claim 11, wherein in response to the receiver coil being concentrically aligned with the transmitter coil while the signal acquisition device is positioned in the dock, an electric current is induced in the receiver coil due to electromagnetic induction so that the rechargeable battery is wirelessly recharged via inductive resonant charging.

13. The biosignal monitoring system of any preceding claims, wherein the dock is mountable to a support structure in a stationary position, and wherein a forwardly-facing surface of the acquisition-device receiver is parallel to the support structure while the dock is mounted to the support structure.

14. The biosignal monitoring system of claim 13, wherein the support structure includes a pole or a wall.

15. The biosignal monitoring system of any preceding claims, wherein the detected biosignals are electrocardiogram signals.
